# EUROPEAN PATENT SPECIFICATION

(11) **EP 3 398 634 B1**
(45) Date of publication and mention of the grant of the patent: **20.04.2022**
(21) Application number: 16881842.5
(22) Date of filing: 28.12.2016
(51) Int. Cl.: A61M 5/303, A61M 5/20, A61M 5/30, A61M 5/24

(54) **ADMINISTRATION APPARATUS**
VORRICHTUNG ZUR VERABREICHUNG
APPAREIL D'ADMINISTRATION

(30) Priority: 28.12.2015 JP 2015255792
(43) Date of publication of application: 07.11.2018
(73) Proprietor: Daicel Corporation, Osaka-shi, Osaka 530-0011 (JP)
(72) Inventor: ODA, Shingo, Osaka-shi, Osaka 530-0011 (JP); NAGAMATSU, Shinji, Osaka-shi, Osaka 530-0011 (JP); YAMASHITA, Kunihiko, Himeji-shi, Hyogo 671-1283 (JP); HASHIMOTO, Aya, Tokyo 108-8230 (JP)
(74) Representative: Jones, Nicholas Andrew
(86) International application number: PCT/JP2016/089190
(87) International publication number: WO 2017/115868

(56) References cited:
- WO-A1-2004/011066
- JP-A- 2013 059 424
- US-A1- 2004 035 491
- US-A1- 2004 133 150
- US-A1- 2005 027 239
- US-A1- 2013 304 017
- US-A1- 2015 032 082

## Description

### [Technical Field]

The present invention relates to an administration apparatus for administering a dosing liquid containing a prescribed substance to an object region.

### [Background Art]

While an injector can be exemplified as an apparatus for administering a drug solution to an object region such as a living organism, needle-free injectors without a needle have been developed in recent years from the perspectives of handleability, hygiene, and the like. Generally, with respect to a needle-free injector, a configuration has been put to practical use in which a drug solution pressurized by a driving source such as compressed gas or a spring is injected toward an object region and the drug solution is administered inside the object region using kinetic energy of the drug solution. Further, the use of powder combustion is being studied as an alternative driving source. As described above, since a needle-free injector does not have a mechanical construction (in other words, a needle) which comes into direct contact with the inside of the object region, needle-free injectors are highly convenient for a user. On the other hand, precisely due to the absence of such a mechanical construction, it is not necessarily easy to administer a drug solution to a desired site within an object region.

PTL 1 discloses a technique for feeding a drug solution to a desired depth of a skin structure of a living organism using a needle-free injector. Specifically, with respect to pressurization for injection of an injection solution, in order to form a through-passage in an injection object region, pressurization control is carried out which involves performing: a first pressurization mode in which, after pressure is raised to first peak pressure, pressure to the injection solution is lowered to stand-by pressure; and a second pressurization mode in which the injection solution at the stand-by pressure is pressurized to raise the pressure to the injection solution to second peak pressure and a prescribed injection amount is injected. Performing such pressurization control controls behavior of the injection solution in the object region.

### [Citation List]

### [Patent Literature]

[PTL 1] Japanese Patent Application Laid-open No. 2012-61269 WO2004/011066 discloses a device and method for liquid jet generation. US2005/027239 discloses an intra dermal injection system for injecting DNA based injectables into humans. US2015/032082 discloses a needleless syringe. US2004/035491 discloses a method and apparatus for needleless injection with a degassed fluid. US2004/133150 discloses a device and method for injecting.

### [Summary of Invention]

### [Technical Problem]

Previously, in administration apparatuses including needle-free injectors which administer various drug solutions, a spring, compressed gas, gunpowder, and the like have been used as a driving source for such administration. Regardless of which driving source is used, a drug solution is pressurized to inject the drug solution to an object region and the drug solution is introduced to the inside of the object region using kinetic energy of the drug solution. Therefore, when the object region is viewed from a microscopic perspective, there is a risk that components of the object region (for example, when the object region is a living organism or the like, tissue, cells, and the like thereof) are subjected to an excessive load, the components are destroyed, and functions thereof are damaged. In particular, in an initial stage of administration to the object region, since the drug solution must penetrate into the object region from the outside, an impact on the object region tends to be significant. When the impact on the object region attributable to the administration is regarded as invasiveness to the object region, such invasiveness is not sufficiently considered in administration apparatuses according to prior art.

For example, with the technique disclosed in PTL 1 described above, preferable injection of an injection solution is achieved by separately controlling pressure applied to the injection solution by powder combustion in two pressurization modes. However, although PTL 1 discloses pressurizing the injection solution at the first peak pressure in an initial stage of injection to enable the injection solution to reach a desired depth, there is no disclosure of performing injection of the injection solution in consideration of invasiveness to the object region. In addition, there are similarly no disclosures of administration control which sufficiently takes invasiveness to an object region into consideration in conventional administration apparatuses other than the needle-free injector according to PTL 1.

In consideration of the problem described above, an object of the present invention is to provide, in an administration apparatus which administers a dosing liquid containing a prescribed substance to an object region, a technique capable of reducing invasiveness to the object region during administration.

### [Solution to Problem]

In order to solve the problem described above, the present invention focuses on a pressure transition of a pressurized dosing liquid in an initial stage of administration. This is due to the fact that, as described earlier, in an administration apparatus, administration to the inside of an object region from the outside is achieved by kinetic energy of the dosing liquid, and components of the object region are subjected to pressure from the dosing liquid particularly when a superficial layer of the object region is penetrated and the inside of the object region is entered in the initial stage of administration.

Specifically, the present invention provides an administration apparatus which administers a dosing liquid containing a prescribed substance to an object region, the administration apparatus including: a main body comprising a through-hole; a storage unit which stores the dosing liquid; a drive unit which imparts administration energy, wherein the drive unit comprises an igniter; a pressurization unit comprising a piston configured to be pressurized by a combustion product generated by the igniter and to slide inside the through-hole, such that the pressurization unit pressurizes the dosing liquid stored in the storage unit with the administration energy in the drive unit; and an injection unit which injects the dosing liquid having been pressurized by the pressurization unit to the object region through an injection port. In addition, the pressurization unit pressurizes the dosing liquid so that, in a pressure transition of the dosing liquid at the injection port in response to imparting the administration energy, a vibration in the pressure transition reaches a converged state within a prescribed period of time from a rising time of pressure wherein the vibration in the pressure transition comprises a first vibration element followed by successive second, third and fourth vibration elements, wherein a ratio of a peak value of the first vibration element in the vibration within the prescribed period of time to a peak value of subsequent vibration elements other than the first vibration element is higher than 1 and equal to or lower than a prescribed first ratio.

In the administration apparatus according to the present invention, the drive unit is configured to generate administration energy used to administer a dosing liquid to an object region. More specifically, the generation of the administration energy may be performed chemically, and an example of the administration energy may be combustion energy which is created by an oxidation reaction of a high-energy substance such as gunpowder or an explosive. In addition, the administration energy may be composite energy which appropriately combines the combustion energy, electrical energy, and internal energy such as elastic energy.

Examples of the high-energy substance include any one of gunpowder containing zirconium and potassium perchlorate, gunpowder containing titanium hydride and potassium perchlorate, gunpowder containing titanium and potassium perchlorate, gunpowder containing aluminum and potassium perchlorate, gunpowder containing aluminum and bismuth oxide, gunpowder containing aluminum and molybdenum oxide, gunpowder containing aluminum and copper oxide, and gunpowder containing aluminum and iron oxide, or gunpowder consisting of a combination of a plurality thereof. With such high-energy substances (gunpowders), since a combustion product thereof characteristically does not have a gaseous component at normal temperature even though the combustion product is gaseous in a high-temperature state, as a result of the combustion product performing condensation immediately after ignition, using such high-energy substances for administration to the object region enables efficient administration to shallower sites of the object region.

The administration energy by the drive unit generates pressurizing force and, as a result, the dosing liquid stored in the storage unit is pushed out and finally injected toward the object region from the injection port. Moreover, in the administration apparatus according to the present invention, the prescribed substance contained in the dosing liquid is exemplified by substances containing a component expected to have efficacy in the object region or a component expected to exhibit a prescribed function in the object region. Therefore, if at least an injection using administration energy generated by the drive unit can be performed, as a physical form of the prescribed substance in the dosing liquid, the prescribed substance may be present in a state of being dissolved in the liquid or in a state of simply being mixed and not dissolved in the liquid. Examples of the prescribed substance to be fed include a vaccine for antibody enhancement, a protein for cosmetic purposes, and cultured cells for hair regrowth, in which case the dosing liquid is formed by having a liquid medium contain the prescribed substance so as to enable the prescribed substance to be injected. As the medium of the dosing liquid, a medium which does not inhibit the efficacy and functionality of the prescribed substance described above after being administered to the inside of the object region is favorable. Alternatively, the medium may enable the efficacy and functionality described above to be exhibited by acting together with the prescribed substance after being administered to the inside of the object region.

In the administration apparatus according to the present invention, pressurization is performed so that a pressure transition of the dosing liquid at the injection port in response to imparting the administration energy or, in other words, a transition of pressure applied by the administration energy to the dosing liquid to be injected (hereinafter, referred to as an "injection pressure transition") is a transition that enables damage to the object region during administration to be mitigated. Moreover, pressure of the dosing liquid at the injection port refers to pressure applied to the dosing liquid immediately after being injected from the injection port or, in other words, pressure applied to the dosing liquid in a vicinity of the injection port and is pressure required by the dosing liquid to be injected from the injection port. Physically, although the pressure applied to the dosing liquid decreases as a distance of separation from the injection port increases due to injection, the present invention focuses on kinetic energy of the dosing liquid at a time point where the dosing liquid is injected from the administration apparatus toward the object region and therefore uses the phrases "pressure of the dosing liquid at the injection port" and a "pressure transition" thereof. Therefore, when there is a prescribed correlation between the distance of separation from the injection port and a pressure drop due to the distance of separation, a pressure transition of the dosing liquid at a position with the distance of separation can be assumed to reflect a "pressure transition of the dosing liquid at the injection port". The injection pressure transition is a pressure transition in an initial stage of administration which begins with the imparting of administration energy. Therefore, the dosing liquid injected by the injection pressure transition undergoes a process of entering the inside of the object region from the outside and subjects the object region to some kind of dynamic action. For this reason, forming the injection pressure transition is extremely important from the perspective of damage mitigation of the object region during administration.

The inventors of the present invention discovered that, in the injection pressure transition, damage to the object region can be significantly mitigated when pressure vibration reaches a converged state within a prescribed period of time from a rising time of pressure and a ratio of a peak value of an initial first vibration element in the vibration within the prescribed period of time to a peak value of subsequent vibration elements other than the first vibration element is higher than 1 and equal to or lower than a prescribed first ratio. Moreover, a vibration element as referred to herein constitutes a part of a pressure vibration included in the injection pressure transition. The inventors of the present invention assume that the damage mitigation is realized by a mechanism described below. However, the inventors of the present invention do not intend to adhere to this mechanism, and consider inventions in which pressurization applied to a dosing liquid by a pressurization unit produces effects similar to those of the present application to fall within the scope of the invention of the present application even if such inventions follow mechanisms that differ from the mechanism which the inventors assume. Specifically, the mechanism of such damage mitigation is conceivably due to the fact that, in a limited period of the prescribed period of time, vibration of pressure of the dosing liquid such that the peak of the first vibration element is greater than those of other vibration elements makes it easier for the dosing liquid to flow into the object region so as to conform to an interface between components of the object region while preferably penetrating a superficial layer of the object region. As a result, effects such as the dosing liquid directly impacting components of the object region can be avoided and, accordingly, invasiveness to the object region during administration may be reduced. In addition, when attempting to introduce the prescribed substance into a cell, by controlling pressure vibration as described above, it is conceivable that a temporary opening of a cell membrane can be created to enable the prescribed substance to be introduced into the cell in an efficient manner.

Therefore, the prescribed first ratio described above is a ratio which enables, in an injection pressure transition, formation of pressure for penetration of a superficial layer of the object region by the dosing liquid, pressure capable of reducing a subsequent dynamic action to the inside of the object region, and the like. The ratio may be appropriately set in accordance with a specific structure (for example, a specific cell or the like of a living organism) or physical characteristics (elastic force) of the object region. In particular, since an excessively large peak value of the first vibration element increases energy of the dosing liquid having flowed into the object region and causes an effect of the energy to the object region to become non-negligible, an upper limit of the ratio may be determined from this perspective. In addition, an excessively long prescribed period of time causes the dosing liquid to vibrate in the object region for a long period of time and may have an adverse effect on an interface between components of the object region. Therefore, the prescribed period of time is a period of 2 msec or less from the rising time and more favorably a period of 1.5 msec or less from the rising time.

In the administration apparatus described above, the first vibration element is a vibration that reaches pressure equal to or higher than 10 MPa and up to approximately 45 MPa that is the peak value within 0.5 msec from the rising time. Causing the first vibration element to steeply rise in this manner suppresses an occurrence of damage at the interface between components of the object region particularly when the dosing liquid penetrates the superficial layer of the object region and enters the inside of the object region.

In addition, in the administration apparatus described heretofore, within the prescribed period of time from the rising time, pressure of the dosing liquid reaches the converged state while vibrating in a damped manner at a frequency belonging to a prescribed frequency range. The prescribed frequency range is 500 to 10000 Hz. Imparting a vibration in a specific prescribed frequency range to the dosing liquid by such pressurization by the pressurization unit makes it easier for the dosing liquid to flow into the object region so as to conform to an interface between components of the object region. This phenomenon conceivably involves a natural frequency or the like of components of the object region.

Furthermore, in the administration apparatus described heretofore, a total amplitude of the second vibration element is approximately 10 MPa, and a total amplitude of the third and fourth vibration elements included in the vibration in the prescribed period of time decreases with the passage of time such that the pressure transition becomes a damped vibration with the passage of time. With respect to the vibration elements other than the first vibration element which penetrates the superficial layer of the object region, invasiveness to the object region can be reduced by preventing the total amplitude of the other vibration elements from becoming excessively large.

Specifically, the inventors of the present invention discovered that damage to the object region can also be significantly mitigated when pressurization by the pressurization unit is performed so that, in the injection pressure transition, pressure vibration dampens over time within a prescribed period of time from a rising time of pressure and, in doing so, the vibration within the prescribed period of time is a damped vibration at a frequency belonging to a prescribed frequency range. The administration energy, the prescribed period of time, and the prescribed frequency range are as described above. The inventors of the present invention assume that the damage mitigation is realized by a mechanism described below. However, the inventors of the present invention do not intend to adhere to this mechanism, and consider inventions in which pressurization applied to a dosing liquid by a pressurization unit produces effects similar to those of the present application to fall within the scope of the invention of the present application even if such inventions follow mechanisms that differ from the mechanism which the inventors assume. Specifically, the mechanism of such damage mitigation is conceivably due to the fact that, in a limited period of the prescribed period of time, the transition of pressure of the dosing liquid while vibrating makes it easier for the dosing liquid to flow into the object region so as to conform to an interface between components of the object region. As a result, effects such as the dosing liquid directly impacting components of the object region can be avoided and, accordingly, invasiveness to the object region during administration may be reduced. In addition, when attempting to introduce the prescribed substance into a cell, by controlling pressure vibration as described above, it is conceivable that a temporary opening of a cell membrane can be created to enable the prescribed substance to be introduced into the cell in an efficient manner.

The administration apparatus which utilizes combustion energy of the high-energy substance described earlier as administration energy may further include a gas generating agent which generates prescribed gas by combustion and which is arranged at a position exposed to combustion products generated by the combustion of the high-energy substance. In addition, the pressurization unit is configured to further pressurize the dosing liquid by energy created by the combustion of the gas generating agent in a vicinity of a time at which the prescribed period of time has elapsed from the rising time of pressure of the dosing liquid. When utilizing combustion energy of a gas generating agent as energy for injecting the dosing liquid, as the gas generating agent, a single-base smokeless powder and various gas generating agents used in an airbag gas generator or a seat-belt pretensioner gas generator can also be used.

When pressurization of the dosing liquid is performed by utilizing a gas generating agent in this manner, favorably, pressure in vicinity of a time at which the prescribed period of time has elapsed from the rising time or, in other words, pressure of the dosing liquid has more or less reached a converged state, or the pressurization is performed at the time when pressure vibration of the dosing liquid has been significantly dampened. Performing such pressurization which utilizes a gas generating agent causes the dosing liquid to be further fed into the object region by combustion of a gas generating agent from a state where the dosing liquid is being fed while reducing invasiveness to the object region by combustion of a high-energy substance. Since a gas generating agent has a relatively slow combustion rate, a pressure transition of the dosing liquid during the combustion of the gas generating agent rises more gradually than during the combustion of the high-energy substance. Therefore, a larger amount of the dosing liquid can be fed to the inside of the object region while maintaining a state where invasiveness to the object region is being reduced.

In addition, in an administration apparatus which uses a gas generating agent in this manner, when the administration energy is defined as first administration energy and energy created by the combustion of the gas generating agent is defined as second administration energy, a peak value in a pressure transition of the dosing liquid by the second administration energy may be equal to or higher than a peak value in a pressure transition excluding the first vibration element in the pressure transition of the dosing liquid by the first administration energy. As described above, the combustion rate of a gas generating agent is relatively gradual. Therefore, even when the peak value in a pressure transition of the dosing liquid by the second administration energy is equal to or higher than the peak value in a pressure transition excluding the first vibration element in a pressure transition by the first administration energy, the object region is sufficiently capable of withstanding a load applied during the pressure transition and is less likely to be damaged. On the other hand, a higher peak value in a pressure transition during the combustion of the gas generating agent enables the dosing liquid to be fed into the object region in an efficient and speedy manner.

The administration apparatus described heretofore may be an apparatus which administers the dosing liquid to the object region from the injection port without involving an introduction unit. Alternatively, the administration apparatus may be a cell processing apparatus which introduces a prescribed substance to cells of a living organism that is an administration object or an apparatus capable of injecting the cells into tissue or an organ of a living organism while performing such processing. Further alternatively, the administration apparatus may be an apparatus which administers a drug solution or the like via a catheter unit to an object region of a living organism. While an administration apparatus according to the present invention will be exemplified as described above, the exemplified administration apparatus is not intended to limit a scope of application of the administration apparatus according to the present invention, and any administration apparatus corresponds to the administration apparatus according to the present invention as long as the administration apparatus includes the invented configurations of the present invention described above.

### [Advantageous Effects of Invention]

In an administration apparatus which administers a dosing liquid containing a prescribed substance to an object region, invasiveness to the object region during administration can be reduced.

### [Brief Description of Drawings]

[Fig. 1]

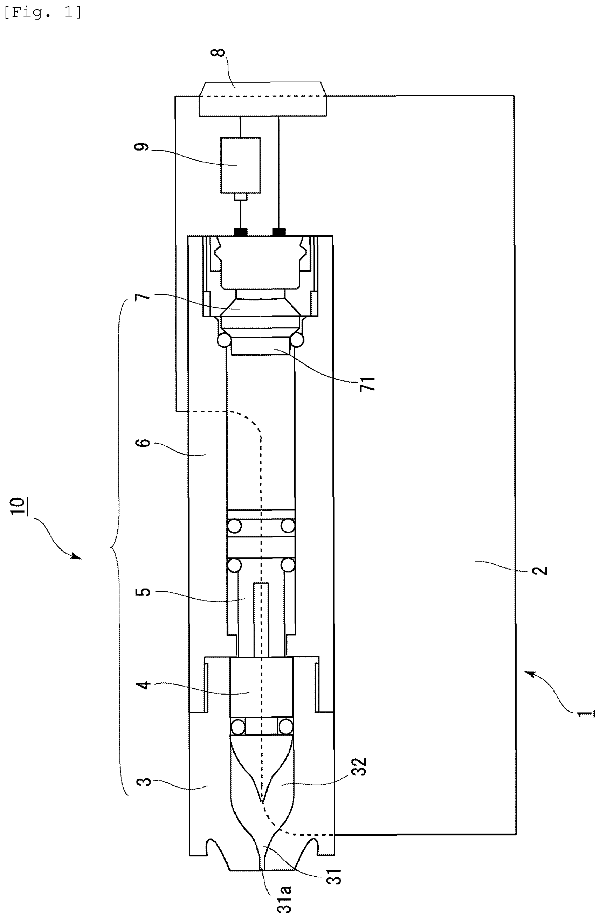

   Fig. 1 is a diagram showing a schematic configuration of an administration apparatus according to the present invention.
[Fig. 2A]

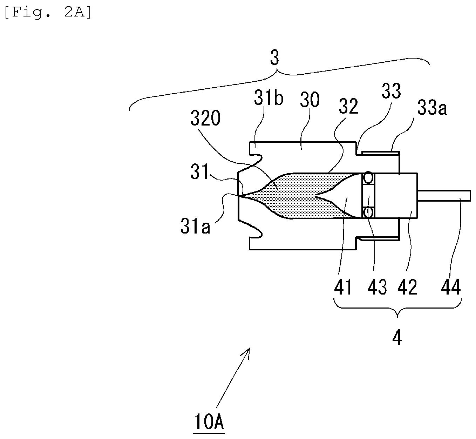

   Fig. 2A is a diagram showing a schematic configuration of a first sub-assembly constituting an apparatus assembly to be built into the administration apparatus shown in Fig. 1.
[Fig. 2B]

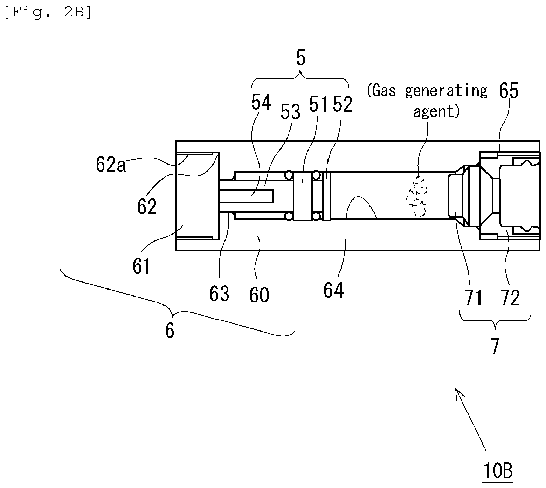

   Fig. 2B is a diagram showing a schematic configuration of a second sub-assembly constituting an apparatus assembly to be built into the administration apparatus shown in Fig. 1.
[Fig. 3]

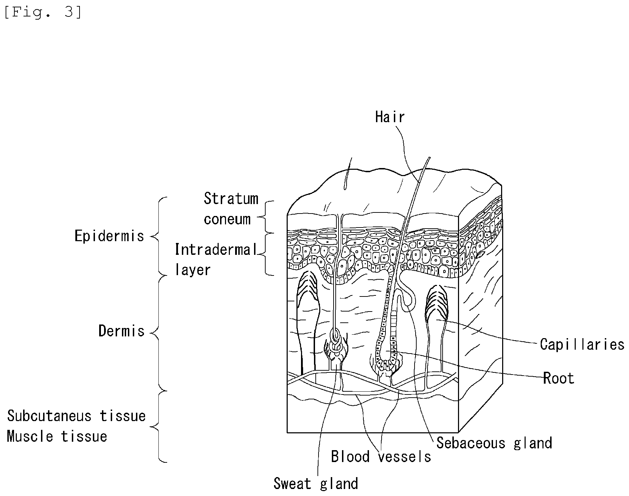

   Fig. 3 is a diagram illustrating a skin structure that is an object region of administration.
[Fig. 4]

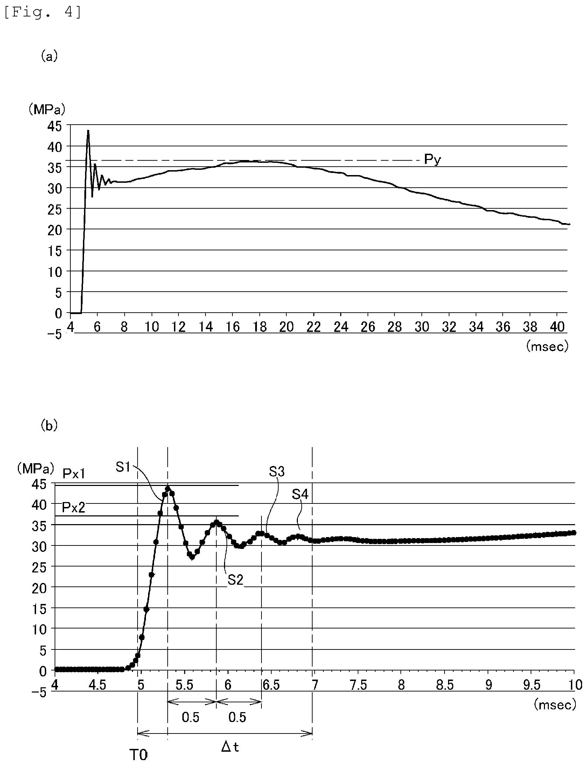

   Fig. 4 is a first diagram showing an injection pressure transition of a dosing liquid injected by the administration apparatus according to the present invention.
[Fig. 5]

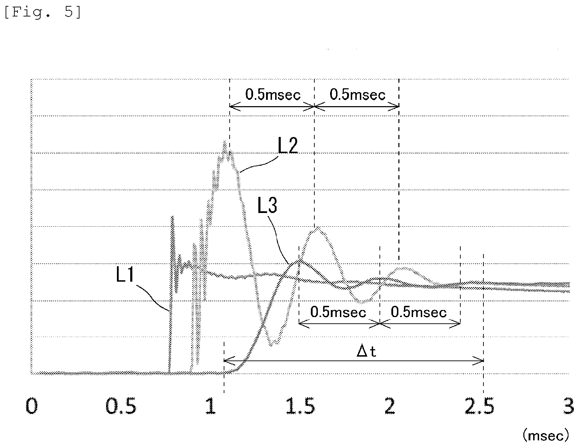

   Fig. 5 is a diagram showing respective transitions of combustion pressure related to powder combustion, pressure applied to a stored dosing liquid, and injection pressure of an injected dosing liquid in the administration apparatus according to the present invention.
[Fig. 6]

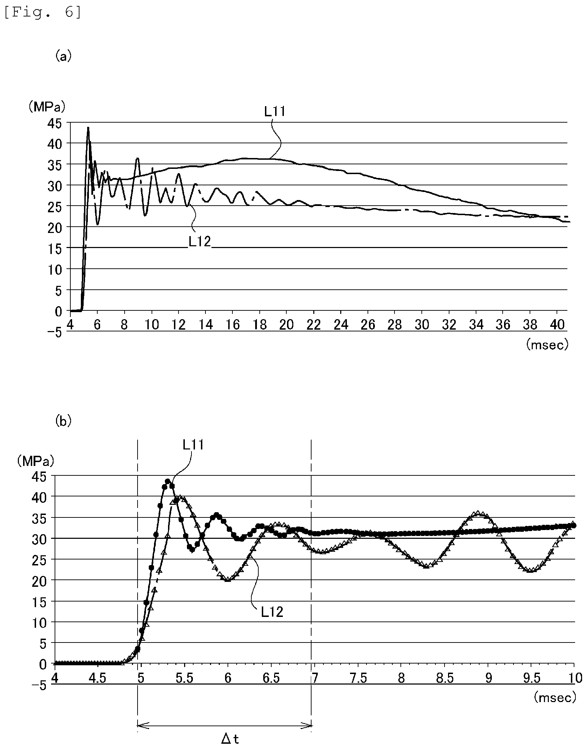

   Fig. 6 is a second diagram showing an injection pressure transition of a dosing liquid injected by the administration apparatus according to the present invention.
[Fig. 7]

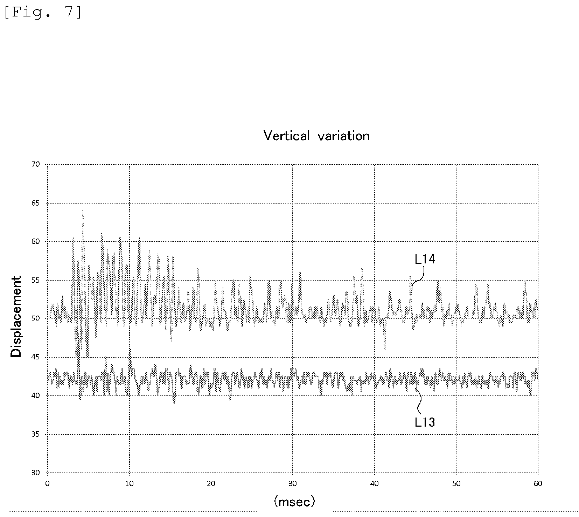

   Fig. 7 is a diagram showing a transition of displacement of a gel in a gel vibration experiment according to a first practical example of the present invention.
[Fig. 8]

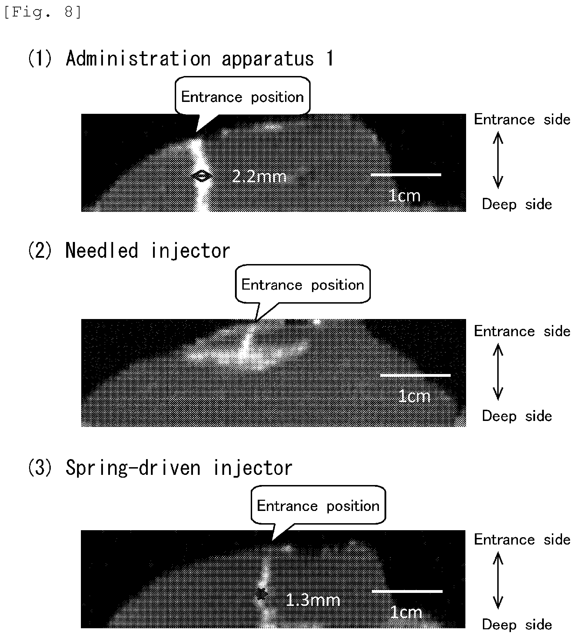

   Fig. 8 is a diagram showing an MRI image representing a result of an administration experiment to porcine abdominal skin tissue according to a third practical example of the present invention.
[Fig. 9]

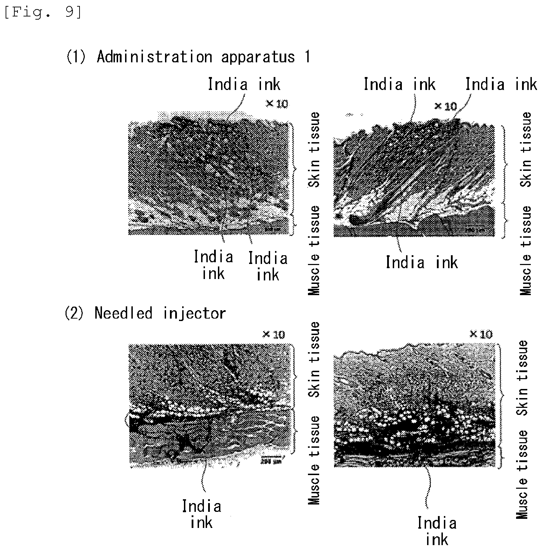

   Fig. 9 is a diagram showing a result of an administration experiment to rat skin tissue according to a fourth practical example of the present invention.

### [Description of Embodiments]

Hereinafter, an administration apparatus 1 according to embodiments of the present invention will be described with reference to the drawings. As described below, the administration apparatus 1 is an apparatus which administers a dosing liquid to an object region, and an administration operation of the dosing liquid corresponds to the administration operation of the administration apparatus according to the present invention. Therefore, the dosing liquid corresponds to the dosing liquid according to the present invention. It is to be understood that configurations of the embodiments described below are illustrative and that the present invention is not limited to the configurations of the embodiments described below. Moreover, in the present embodiment, a "distal end side" and a "proximal end side" will be used as terms representing a relative positional relationship in a longitudinal direction of the administration apparatus 1. The "distal end side" represents a position near to a distal end of the administration apparatus 1 to be described later or, in other words, a position near to an injection port 31a, and the "proximal end side" represents a direction opposite to the "distal end side" in the longitudinal direction of the administration apparatus 1 or, in other words, a direction of a side of a drive unit 7.

### <Configuration of administration apparatus 1>

Fig. 1 is a diagram showing a schematic configuration of the administration apparatus 1 and is also a sectional view along a longitudinal direction of the administration apparatus 1. The administration apparatus 1 is constructed by mounting, to a housing 2, an apparatus assembly 10 in which a sub-assembly (refer to Fig. 2A to be described later) 10A which is constituted by a syringe section 3 and a plunger 4 to be described later and a sub-assembly (refer to Fig. 2B to be described later) 10B which is constituted by an administration apparatus main body 6, a piston 5, and the drive unit 7 are integrally assembled. In the following description of the present application, a dosing liquid that is administered to an object region by the administration apparatus 1 is formed by having a liquid medium contain a prescribed substance which exhibits expected efficacy or a function in the object region. In the dosing liquid, the prescribed substance may be in a state of being dissolved in a liquid that is the medium or in a state of being simply mixed without being dissolved.

Examples of the prescribed substance contained in the dosing liquid include not only drugs and medicines which can be administered in the object region that is a living organism but also biologically-derived substances and substances which produce desired physiological activity, examples of the biologically-derived substances include DNA, RNA, nucleic acids, antibodies, cells, and examples of the physiologically active substances include low molecular medicine, inorganic material such as metal particles for hyperthermia and radiotherapy, and substances and the like having various pharmacological and therapeutic effects including carriers. In addition, the liquid that is the medium of the dosing liquid need only be a substance preferable for administering the prescribed substances into the object region and may be either water-based or oil-based. Furthermore, as long as the prescribed substance can be administered with the administration apparatus 1, a viscosity of the liquid that is the medium is also not particularly limited. In addition, the object region that is an administration object of the dosing liquid is a region to which the prescribed substance is to be administered, and examples of the object region include cells or tissue (skin or the like), an organ or system (an eye, the heart, a liver, or the like), and the like of a living organism. Moreover, a component of a living organism can be set as the object region in a state where the component is separated from a main body of the living organism as long as such separation does not pose any problems. In other words, both ex-vivo administration of the prescribed substance to an object region (tissue or an organ) and in-vitro administration of the prescribed substance to an object region (cultured cells or cultured tissue) are also included in the scope of the administration apparatus according to the present invention.

As described above, the apparatus assembly 10 is configured to be detachable from and attachable to the housing 2. A storage chamber 32 (refer to Fig. 2A) formed between the syringe section 3 and the plunger 4 included in the apparatus assembly 10 is filled with the dosing liquid, and the apparatus assembly 10 is a unit which is replaced every time an injection of the dosing liquid is performed. On the other hand, a battery 9 that supplies power to an igniter 71 included in the drive unit 7 of the apparatus assembly 10 is included on a side of the housing 2. Power supply from the battery 9 is performed via a wiring between an electrode on the side of the housing 2 and an electrode on a side of the drive unit 7 of the apparatus assembly 10 when a user performs an operation that involves pressing down a button 8 provided on the housing 2. Moreover, shapes and positions of the electrode on the side of the housing 2 and the electrode on the side of the drive unit 7 of the apparatus assembly 10 are designed so that the electrodes automatically come into contact with each other when the apparatus assembly 10 is mounted to the housing 2. In addition, the housing 2 is a unit which can be repetitively used as long as power that can be supplied to the drive unit 7 remains in the battery 9. Furthermore, in the housing 2, when power of the battery 9 is used up, only the battery 9 may be replaced and the housing 2 may be continuously used.

Configurations of the sub-assemblies 10A and 10B and detailed configurations of the syringe section 3, the plunger 4, the piston 5, the administration apparatus main body 6, and the drive unit 7 which are included in both sub-assemblies will now be described with reference to Figs. 2A and 2B. The syringe section 3 has a nozzle section 31 which includes the storage chamber 32 that is a space capable of storing the dosing liquid and, at the same time, the plunger 4 is arranged in the sub-assembly 10A so as to be slidable in the storage chamber 32.

As a body 30 of the syringe section 3, for example, known nylon 6-12, polyallylate, polybutylene terephthalate, polyphenylene sulfide, a liquid crystal polymer, or the like can be used. In addition, these resins may contain a filler such as glass fiber or a glass filler, in which case polybutylene terephthalate can contain 20% by mass to 80% by mass of glass fiber, polyphenylene sulfide can contain 20% by mass to 80% by mass of glass fiber, and a liquid crystal polymer can contain 20% by mass to 80% by mass of minerals.

In addition, in the storage chamber 32 formed inside the body 30, the plunger 4 is arranged so as to be slidable in a direction of the nozzle section 31 (a distal end-side direction), and a space formed between the plunger 4 and the body of the syringe section 3 is a space in which a dosing liquid 320 is to be stored. As the plunger 4 slides inside the storage chamber 32, the dosing liquid 320 stored in the storage chamber 32 is pressed and injected from the injection port 31a provided on a distal end side of the nozzle section 31. To this end, the plunger 4 is formed of a material which enables the plunger 4 to slide smoothly in the storage chamber 32 and which prevents the dosing liquid 320 from leaking out from the side of the plunger 4. Specific examples of a material of the plunger 4 include butyl rubber and silicone rubber. Further examples include a styrene-based elastomer, a hydrogenated styrene-based elastomer, and a styrene-based elastomer or a hydrogenated styrene-based elastomer mixed with a polyolefin such as polyethylene, polypropylene, polybutene, or alpha-olefin copolymer, an oil such as liquid paraffin or processing oil, or a powdered inorganic substance such as talc, cast, or mica. In addition, a polyvinyl chloride-based elastomer, an olefin-based elastomer, a polyester-based elastomer, a polyamide-based elastomer, a polyurethane-based elastomer, various rubber materials (in particular, vulcanized rubber materials) such as natural rubber, isoprene rubber, chloroprene rubber, nitrilebutadiene rubber, styrene-butadiene rubber, and mixtures thereof can also be adopted as the material of the plunger 4. Furthermore, for the purpose of securing or adjusting slidability between the plunger 4 and the syringe section 3, a surface of the plunger 4 or a surface of the storage chamber 32 of the syringe section 3 may be coated or surface-treated using various substances. As the coating agent, PTFE (polytetrafluoroethylene), silicone oil, diamond-like carbon, nanodiamond, and the like can be used, and an injection pressure transition and vibration elements thereof to be described later may be controlled by using these material to adjust slidability to a desired range.

As shown in Fig. 2A, the plunger 4 has a head section 41 and a barrel section 42, and the head section 41 and the barrel section 42 are connected by a neck section 43 with a diameter that is smaller than diameters of the head section 41 and the barrel section 42. The diameter of the neck section 43 is made small in this manner in order to form a storage space of an O ring that constitutes a seal member. Moreover, a contour on a distal end side of the head section 41 has a shape that more or less conforms to a contour of an inner wall surface of the nozzle section 31. Accordingly, when the plunger 4 slides to the side of the nozzle section 31 during injection of the dosing liquid and reaches a deepest position that is an innermost position in the storage chamber 32, a gap formed between the plunger 4 and the inner wall surface of the nozzle section 31 can be made as small as possible and the dosing liquid 320 can be prevented from remaining inside the storage chamber 32 and becoming waste. However, the shape of the plunger 4 is not limited to any particular shape as long as a desired effect can be obtained with the administration apparatus according to the present invention. In addition, an injection pressure transition and vibration elements thereof to be described later can be controlled by providing a single protrusion or a plurality of protrusions in the barrel section 42 and adjusting a contact area between the plunger 4 and the storage chamber 32 or varying a shape of the protrusions in order to adjust the slidability between the plunger 4 and the syringe section 3.

Furthermore, the plunger 4 is provided with a rod section 44 which extends further in a direction of the proximal end side from an end surface on the proximal end side of the barrel section 42. While a diameter of the rod section 44 is sufficiently smaller than that of the barrel section 42, the diameter is large enough to enable the user to grip the rod section 44 and move the rod section 44 inside the storage chamber 32. In addition, a length of the rod section 44 is determined so that the rod section 44 protrudes from the end surface on the proximal end side of the syringe section 3 and the user can grip the rod section 44 even when the plunger 4 is at the deepest position of the storage chamber 32 of the syringe section 3.

Let us now return to the description of the syringe section 3. An inner diameter of a flow path provided in the nozzle section 31 on the side of the syringe section 3 is formed narrower than an inner diameter of the storage chamber 32. According to such a configuration, the dosing liquid 320 having been pressurized to high pressure is injected to the outside from the injection port 31a of the flow path. In consideration thereof, a vicinity of the nozzle section 31 on a distal end side of the syringe section 3 is provided with an annular shield section 31b so as to surround a periphery of the injection port 31a. For example, when the dosing liquid is to be injected by pressing the injection port 31a against a superficial layer of an administration object region such as human skin, the injected dosing liquid can be shielded by the shield section 31b and prevented from spattering around the injection port 31a. Moreover, a certain amount of yielding of the skin when the injection port is pressed against the skin increases contactability between the injection port and the skin and can suppress scattering of the dosing liquid. In consideration thereof, as shown in Fig. 2A, a distal end of the nozzle section 31 where the injection port 31a is positioned may slightly protrude in an injection direction of the dosing liquid from the end surface of the shield section 31b.

In addition, in the neck section 33 that is positioned on the proximal end side of the syringe section 3, a screw section 33a for coupling the administration apparatus main body 6 on the side of the sub-assembly 10B to be described later and the syringe section 3 to each other is formed. A diameter of the neck section 33 is set smaller than the diameter of the body 30.

Next, the sub-assembly 10B that includes the piston 5, the administration apparatus main body 6, and the drive unit 7 will be described with reference to Fig. 2B. The piston 5 is configured to be pressurized by a combustion product generated by the igniter 71 of the drive unit 7 and to slide inside a through-hole 64 formed inside a body 60 of the administration apparatus main body 6. In the administration apparatus main body 6, a coupling depression 61 is formed on a distal end side with the through-hole 64 as a reference. The coupling depression 61 is a portion which is coupled with the neck section 33 of the syringe section 3 described above, and a screw section 62a which screws with the screw section 33a provided in the neck section 33 is formed on a side wall surface 62 of the coupling depression 61. In addition, while the through-hole 64 and the coupling depression 61 are connected by a communicating section 63, a diameter of the communicating section 63 is set smaller than a diameter of the through-hole 64. Furthermore, in the administration apparatus main body 6, a drive unit depression 65 is formed on a proximal end side with the through-hole 64 as a reference. The drive unit 7 is to be arranged in the drive unit depression 65.

In addition, the piston 5 is made of metal and has a first barrel section 51 and a second barrel section 52. The piston 5 is arranged inside the through-hole 64 so that the first barrel section 51 faces a side of the coupling depression 61 and, at the same time, the second barrel section 52 faces a side of the drive unit depression 65. The piston 5 slides inside the through-hole 64 of the administration apparatus main body 6 while the first barrel section 51 and the second barrel section 52 oppose an inner wall surface of the through-hole 64. Moreover, the first barrel section 51 and the second barrel section 52 are connected by a coupling section that is narrower than diameters of the respective barrel sections, and an O ring or the like is arranged in a space between the barrel sections which is formed as a result of the barrel sections being connected by the coupling section in order to enhance adhesiveness with the inner wall surface of the through-hole 64. In addition, the piston 5 may be made of resin, in which case metal may be used in combination with the resin in portions that require heat resistance or pressure resistance.

An end surface on a distal end side of the first barrel section 51 is provided with a pressing column section 53 of which a diameter is smaller than the first barrel section 51 and also smaller than the diameter of the communicating section 63 of the administration apparatus main body 6. The pressing column section 53 is provided with a storage hole 54 which opens on an end surface on a distal end side of the pressing column section 53, of which a diameter is equal to or larger than the diameter of the rod section 44, and of which a depth is greater than the length of the rod section 44. As a result, when the piston 5 is pressurized by a combustion product of the igniter 71, the pressing column section 53 can transfer the combustion energy of the combustion product to the end surface on the proximal end side of the barrel section 42 of the plunger 4 via the end surface on the distal end side of the pressing column section 53. Moreover, a shape of the piston 5 is similarly not limited to the shape illustrated in Fig. 2B.

Next, the drive unit 7 will be described. The drive unit 7 has a body 72 thereof formed in a cylindrical shape and has, inside the drive unit 7, the igniter 71 which is an electric igniter that burns an ignition charge to generate energy for injection, and the drive unit 7 is arranged in the drive unit depression 65 as described above so as to be capable of transferring combustion energy by the igniter 71 to the second barrel section 52 of the piston 5. Specifically, the body 72 of the drive unit 7 may be constructed by fixing an injection-molded resin to a metal collar. Known methods can be used to perform the injection molding. A resin material of the body 72 of the drive unit 7 is the same resin material used to form the body 30 of the syringe section 3.

The ignition charge used in the igniter 71 corresponds to the high-energy substance according to the present invention. In addition, the combustion energy of the ignition charge corresponds to the administration energy according to the present invention and, consequently, the generation of the combustion energy or, in other words, the combustion of the ignition charge corresponds to the imparting of the administration energy. Favorable examples of the ignition charge include gunpowder (ZPP) containing zirconium and potassium perchlorate, gunpowder (THPP) containing titanium hydride and potassium perchlorate, gunpowder (TiPP) containing titanium and potassium perchlorate, gunpowder (APP) containing aluminum and potassium perchlorate, gunpowder (ABO )containing aluminum and bismuth oxide, gunpowder (AMO) containing aluminum and molybdenum oxide, gunpowder (ACO) containing aluminum and copper oxide, and gunpowder (AFO) containing aluminum and iron oxide, and gunpowder consisting of a combination of a plurality of these gunpowders. These gunpowders exhibit characteristics in that, while high-temperature and high-pressure plasma is generated during combustion immediately after ignition, generated pressure drops abruptly once a combustion product reaches normal temperature and condenses since the combustion product does not have a gaseous component. Gunpowders other than the above may be used as the ignition charge insofar as appropriate administration can be performed.

In addition, although a gas generating agent is not particularly arranged inside the administration apparatus main body 6 shown in Fig. 1 as an additional gunpowder component, in order to adjust a pressure transition applied to the dosing liquid via the piston 5, a gas generating agent or the like which is burned by the combustion product created by powder combustion in the igniter 71 to generate gas can also be arranged inside the administration apparatus main body 6. An arrangement location of the gas generating agent is a location where the gas generating agent may be exposed to the combustion product from the igniter 71 such as a location depicted by a dotted line in Fig. 2B. The gas generating agent to be arranged inside the igniter 71 is already well known as disclosed in WO 2001/031282 and Japanese Patent Application Laid-open No. 2003-25950. In addition, examples of the gas generating agent include a single-base smokeless powder consisting of 98% by mass of nitrocellulose, 0.8% by mass of diphenylamine, and 1.2% by mass of potassium sulfate. Furthermore, various gas generating agents used in an airbag gas generator or a seat-belt pretensioner gas generator can also be used. By adjusting dimensions or a size, a shape, and particularly a surface profile of a gas generating agent when arranging the gas generating agent inside the through-hole 64, a combustion completion time of the gas generating agent can be varied and, accordingly, a pressure transition applied to the dosing liquid can be adjusted and a desired injection pressure transition of the dosing liquid can be achieved. In the present invention, the gas generating agent used when necessary is also included in the drive unit 7.

Moreover, filling of the dosing liquid 320 in the sub-assembly 10A is performed in a state where the plunger 4 has been inserted to the deepest position by immersing the injection port 31a into a container filled with the dosing liquid and drawing back the plunger 4 to an opening side of the storage chamber 32 or, in other words, the proximal end side of the syringe section 3 while maintaining this state. At this point, the plunger 4 has been drawn out until an end surface on the proximal end side of the barrel section 42 of the plunger 4 reaches a position slightly protruding from the end surface on the proximal end side of the syringe section 3.

In addition, in the sub-assembly 10B, the piston 5 is first inserted from the proximal end side of the administration apparatus main body 6 shown in Fig. 2B. In doing so, the piston 5 is inserted into the through-hole 64 so that the pressing column section 53 faces the side of the coupling depression 61. Furthermore, the piston 5 is positioned so that an end surface on the distal end side of the piston 5 or, in other words, an end surface on the distal end side of the pressing column section 53 on which the storage hole 54 opens protrudes by a prescribed amount from a bottom surface of the coupling depression 61 (a surface perpendicular to the side wall surface 62). The piston 5 may be positioned by appropriately using known techniques such as marking a symbol for positioning inside the through-hole 64 and using a positioning jig. Subsequently, the drive unit 7 is mounted to the drive unit depression 65. Moreover, a fixing force of the piston 5 in the through-hole 64 is set depending on pressure received by the drive unit 7 from a combustion product by the igniter 71 to a level which enables the piston 5 to slide inside the through-hole 64 in a sufficiently smooth manner and, at the same time, a level which sufficiently counteracts a force received by the piston 5 from the plunger 4 when the sub-assembly 10A is mounted to the sub-assembly 10B and which prevents the position of the piston 5 from fluctuating.

The sub-assembly 10A configured in this manner is mounted to the sub-assembly 10B by screwing of the screw sections 33a and 62a to form the apparatus assembly 10. At this point, as coupling of both sub-assemblies advances, the rod section 44 of the plunger 4 enters the storage hole 54 provided in the pressing column section 53 of the piston 5 and becomes stored in the storage hole 54 and, finally, the end surface on the distal end side of the pressing column section 53 comes into contact with the end surface on the proximal end side of the barrel section 42 of the plunger 4. Moreover, since the storage hole 54 is large enough to store the rod section 44, in this contact state, an inner wall surface at the back of the storage hole 54 (in particular, a bottom surface of the storage hole 54) is not in contact with the end section on the proximal end side of the rod section 44 and, therefore, the rod section 44 is not subjected to a load from the side of the piston 5. Furthermore, once the coupling advances to a final screwing position, since a position of the piston 5 is fixed to the through-hole 64 with a sufficient friction force, the plunger 4 is pushed by the pressing column section 53 so as to advance toward the side of the injection port 31a and the plunger 4 is positioned inside the syringe section 3. Moreover, a part of the dosing liquid 320 corresponding to the amount by which the plunger 4 is pushed is discharged from the injection port 31a.

The formation of the apparatus assembly 10 is completed once the plunger 4 is positioned at a final position in this manner. In the apparatus assembly 10, the piston 5 is in a state of being positioned at a prescribed position with respect to the administration apparatus main body 6, and a position of the plunger 4 in the storage chamber 32 of the syringe section 3 is finally mechanically determined with the piston 5 as a reference. Since the final position of the plunger 4 is a position that is uniquely determined in the apparatus assembly 10, an amount of the dosing liquid 320 to be finally stored in the storage chamber 32 can be adjusted to a prescribed amount determined in advance.

Subsequently, the apparatus assembly 10 is mounted to the housing 2, and when the button 8 is pressed by the user in a state where the injection port 31a is in contact with the object region, the dosing liquid 320 is pressurized via the piston 5 and the plunger 4, injection of the dosing liquid 320 is executed, and the dosing liquid 320 is administered into the object region.

### <Injection control of dosing liquid>

Examples of the object region of the administration apparatus 1 include a skin structure of a living organism such as a human or livestock. Fig. 3 schematically shows an anatomical structure of human skin. The human skin is structured in layers in the depth direction from the skin surface side by the epidermis, the dermis, and subcutaneous tissue and muscle tissue, and the epidermis can be further divided into the layers of the stratum corneum and the intradermal layer. The respective layers of the skin structure differ from each other in cells and the like which mainly make up tissue of the layer and in tissue characteristics.

Specifically, the stratum corneum is mainly made up of keratinocytes and functions as a so-called barrier layer due to its position on an outermost side of skin. Generally, a thickness of the stratum corneum is around 0.01 to 0.015 mm and the keratinocytes provide surface protection for a human. Therefore, the stratum corneum is required to be relatively strong in order to physically cut off the inside of a human body from the external environment. In addition, the intradermal layer is made up of dendritic cells (Langerhans cells) and pigment cells (melanocytes), the epidermis is made up of the stratum corneum and the intradermal layer, and a thickness of the epidermis is generally around 0.1 to 2 mm. The dendritic cells in the intradermal layer are thought to be cells involved in antigen-antibody reactions. This is because ingesting an antigen makes the dendritic cells aware of the presence of the antigen and an antigen-antibody reaction which activates lymphocytes that attack foreign objects is more readily induced. On the other hand, pigment cells in the intradermal layer have a function of preventing the effect of ultraviolet rays irradiated from the external environment. In addition, the dermis is lined with cutaneous blood vessels and capillaries that form a complex pattern, and sweat glands for regulating body temperature, roots of body hair (including head hair) and sebaceous glands that accompany roots are also present in the dermis. Furthermore, the dermis is a layer that communicates between the inside of the human body (subcutaneous tissue, muscular tissue) and the epidermis, and is constructed so as to contain fibroblasts and collagen cells. Therefore, a state of the dermis plays a major role in the occurrence of so-called wrinkles, hair loss, and the like due to a shortage of collagen and elastin.

As described above, the skin structure of a human is generally formed in layers and unique anatomical functions are exhibited by cells, tissue, and the like which primarily make up each layer. This means that, when using medical treatment or the like on skin, a substance for the treatment is desirably administered to a location (depth) of the skin structure corresponding to a therapeutic purpose of the medical treatment. For example, since dendritic cells are present in the intradermal layer, a more effective antigen-antibody reaction can be expected by administering a vaccine to the intradermal layer. In addition, since pigment cells are present in the intradermal layer, even when performing a beauty treatment for so-called skin whitening, prescribed substances for skin whitening are required to be administered to the intradermal layer. Furthermore, since fibroblasts and collagen cells are present in the dermis, an effective beauty treatment effect is expected when injecting proteins, enzymes, vitamins, amino acids, minerals, sugars, nucleic acids, and various growth factors (epithelial cells and fibroblasts) for removing wrinkles of the skin. Moreover, it is said that a favorable hair regrowth treatment involves stem cell infusion in which hair papilla cells, epidermal stem cells, and the like are autologously cultured and autologously transplanted into the scalp or injecting several types of growth factors and nutritional components extracted from stem cells into a vicinity of the dermis.

As described above, while a prescribed substance administered according to a therapeutic purpose and a position (depth) in the skin structure at which the prescribed substance is desirably administered individually correspond to each other, it is not easy to deliver the substance to a targeted arrival position. In addition, even when the prescribed substance is able to reach the target arrival position, a sufficient desired effect by the prescribed substance cannot be expected if cells in the vicinity of the arrival position end up being destroyed by a dosing liquid including the prescribed substance. Furthermore, if the dosing liquid had applied some kind of load to tissue and cells through which the dosing liquid had passed in the process of reaching the arrival position and has damaged or destroyed the tissue or cells, such damage or destruction is to be recognized by the user as an internal hemorrhage, pain, and the like and therefore impart a feeling of discomfort to the user. In particular, when no structure (introduction unit) which guides the dosing liquid to the inside of an object region is present between an apparatus to administer the dosing liquid such as the administration apparatus 1 and the object region, a constant amount of energy (in the case of the present invention, this energy is combustion energy by the igniter 71) is to be imparted to the dosing liquid to enable the dosing liquid to penetrate to the inside of the object region. Since the dosing liquid is imparted with relatively high energy and injected toward the object region to enable the dosing liquid to penetrate into the object region, an unnecessary dynamic action is likely to be performed on components (for example, cells) of the object region and invasiveness to the object region cannot be necessarily described as being low. In addition, in conventional art, injections of a dosing liquid which sufficiently take such invasiveness to the object region into consideration are not carried out and, consequently, efficacy and the like due to the prescribed substance are not sufficiently elicited.

In consideration thereof, in the present invention, for example, minimal invasiveness to an object region is defined as administering a dosing liquid so that functions of organs, tissue, and the like of a living organism are not damaged during administration or administering the dosing liquid so that damage to such functions are minimized. Alternatively, when cells of a living organism are used as an object region, minimal invasiveness to an object region is defined as administering a dosing liquid so as not to cause unnecessary cell death or administering the dosing liquid so as to minimize unnecessary cell death. Based on the above, the administration apparatus 1 according to the present invention adopts a configuration in which pressurization of a dosing liquid by combustion energy generated by the drive unit 7 is adjusted so that a pressure transition of the dosing liquid injected from the administration apparatus 1 exhibits minimal invasiveness with respect to an object region. Details thereof will be described below.

Fig. 4 is a diagram showing a transition of pressure (hereinafter, simply referred to as "injection pressure") of a dosing liquid injected from the injection port 31a when the dosing liquid is injected by driving of the drive unit 7 in the administration apparatus 1. In Fig. 4, an abscissa represents elapsed time in milliseconds and an ordinate represents injection pressure in MPa. Moreover, injection pressure can be measured using conventional art. For example, in a similar manner to a measurement method described in Japanese Patent Application Laid-open No. 2005-21640, an injection force can be measured by a method involving applying force of an injection in a distributed manner to a diaphragm of a load cell arranged on a downstream side of a nozzle, sampling output from the load cell with a data sampling apparatus via a detection amplifier, and storing the sampled output as an injection force (N) per unit time. Injection pressure is calculated by dividing an injection force measured in this manner by an area (a nozzle area) of the injection port 31a of the administration apparatus 1. The example shown in Fig. 4 represents a transition of injection pressure obtained by adopting ZPP (containing zirconium and potassium perchlorate) as an ignition charge of the igniter 71 inside the drive unit 7 and, at the same time, arranging a gas generating agent inside the through-hole 64.

In Fig. 4, an upper part (a) thereof represents a transition of injection pressure during a period of approximately 40 milliseconds from start of combustion with a time point at which the button 8 is pressed in the drive unit 7 as an origin, and a lower part (b) thereof displays an enlargement of an injection pressure transition in an initial period (approximately 10 milliseconds from the origin) in the pressure transition shown in the upper part (a). Moreover, rising of injection pressure occurs not at the origin but in a vicinity of 5 milliseconds because a certain amount of time is required for the ignition charge to burn, the dosing liquid to be pressurized as the piston 5 is propelled by the combustion energy of the ignition charge, and the dosing liquid to be injected from the injection port 31a. As is apparent from Fig. 4, in the injection pressure transition, a plurality of pressure vibration elements S1 to S4 are present in a prescribed period of time Δt from the rise timing T0 to approximately 2 milliseconds thereafter, and pressure vibration generally converges once the prescribed period of time Δt elapses. Moreover, in the present invention, one cycle in which injection pressure rises and drops in pressure vibration is to be handled as one pressure vibration element.

More specifically, in the prescribed period of time Δt from the rise timing T0, a pressure vibration element S1 (hereinafter, referred to as a "first vibration element S1") is initially generated. The first vibration element S1 is an injection pressure transition of a period after a peak value P×1 (approximately 45 MPa) once arrives from injection pressure (approximately 0 MPa) at the rise timing T0 and before a next local minimum value arrives. In addition, a variation width (peak to peak) of the injection pressure in the period is defined as a total amplitude of the first vibration element S1 and, specifically, the total amplitude of the first vibration element S1 is approximately 45 MPa. The first vibration element S1 is further followed by a second vibration element S2, a third vibration element S3, and a fourth vibration element S4. The second vibration element S2 is an injection pressure transition of a period after a peak value P×2 (approximately 37 MPa) arrives from a timing of the end of the first vibration element S1 and before a next local minimum value arrives. In addition, a variation width (peak to peak) of the injection pressure in the period is defined as a total amplitude of the second vibration element S2 and, specifically, the total amplitude of the second vibration element S2 is approximately 10 MPa. With respect to the third vibration element S3 and the fourth vibration element S4, a period that defines each vibration element and a total amplitude of each vibration element are similar to those of the second vibration element S2 and, although a detailed description thereof will be omitted, the total amplitude of the third vibration element S3 and the total amplitude of the fourth vibration element S4 have decreased with the passage of time. In other words, in the prescribed period of time Δt, the pressure transition becomes a damped vibration with the passage of time, and after the lapse of the prescribed period of time Δt, the pressure transition enters a state where the vibration has more or less converged.

Furthermore, let us focus on a period of the pressure vibration in the prescribed period of time Δt. A period calculated from a peak value of the first vibration element S1 and a peak value of the second vibration element S2 is approximately 0.5 milliseconds, and a period calculated from the peak value of the second vibration element S2 and a peak value of the third vibration element S3 is approximately 0.5 milliseconds. While a period immediately before arrival of a converged state is slightly shorter, it is evident that the transition of injection pressure takes place at a generally constant period in the prescribed period of time Δt. Accordingly, it is shown that the injection pressure transition in the prescribed period of time Δt is a pressure vibration at a frequency of around 2000 Hz.

Moreover, a pressure fluctuation in the prescribed period of time Δt is mainly attributable to combustion of the ignition charge of the igniter 71. In addition, in a vicinity of a timing at which the prescribed period of time Δt lapses, combustion of the gas generating agent inside the through-hole 64 is started by a combustion product of the ignition charge and combustion energy thereof starts to further act on the dosing liquid. As a result, as shown in Fig. 4(a), after the lapse of the prescribed period of time Δt, injection pressure increases one again and a peak value Py arrives at a timing of approximately 18 milliseconds. Moreover, subsequently, the injection pressure gradually drops with the passage of time. Since a combustion rate of the gas generating agent is lower than a combustion rate of the ignition charge, a rate of increase of injection pressure due to combustion of the gas generating agent also becomes relatively lower.

How a dosing liquid affects components of an object region and exhibits minimal invasiveness due to injection pressure indicating such transition characteristics will be described using an example of administrating the dosing liquid to the epidermis of the skin structure shown in Fig. 3. Various bonding means are present in basal cells in the stratum basale of the epidermis. The epidermis and the dermis are bonded in close contact by an epidermal basement membrane structure, and a portion where the epidermis and the dermis are in contact with each other is called an epidermal basement membrane. A transparent band is present between a cell membrane and the basal lamina of basal cells, and hemidesmosomes play a major role in the bonding between the basal cells and the basement membrane structure. In addition, desmosomes and gap junctions are involved in the bonding of adjacent basal cells. A desmosome has a portion on an inner side of the cell membrane called an attachment plaque and a structure responsible for bonding between cells through the cell membrane. Furthermore, a gap junction is formed by connexins and is structured so as to bond adjacent cells across a gap of 2 to 3 nm. Due to these bonding means, prevention of separation of the epidermis and the dermis from one another and retention of moisture in the skin structure are achieved.

As described above, in the skin structure of a living organism, cells themselves are enclosed in the cell membrane and, at the same time, various bonding means are present between the cells. Therefore, in order to demonstrate minimal invasiveness to cells and the like when the skin structure is used as an object region of administration of a dosing liquid, control of injection pressure which suitably counteracts a bonding force between cells created by the bonding means while avoiding unnecessary dynamic action to cell membranes is conceivably important. In addition, technical concepts related to such injection pressure are conceivably applicable in a similar manner to object regions in a living organism other than the skin structure.

In the injection pressure transition shown in Fig. 4(b), within the prescribed period of time Δt, the first vibration element S1 with highest peak pressure is initially generated, followed by the sequential arrivals of the second, third, and fourth vibration elements S2 to S4. Through this process, the total amplitude of the respective vibration elements gradually decreases and pressure vibration is dampened. Causing injection pressure to vibrate in a damped manner in the prescribed period of time Δt as described above enables the dosing liquid having been imparted with relatively high energy to enable the dosing liquid to penetrate a superficial layer of the object region in an initial stage of administration to exert a dynamic action on cell membrane in a distributed manner and conceivably enables destruction of cell membrane to be avoided. In particular, as described above, it is shown that the injection pressure transition in the prescribed period of time Δt is a pressure vibration at a frequency of around 2000 Hz which is relatively close to a natural frequency of cells of a living organism. Therefore, when a dosing liquid having the injection pressure transition shown in Fig. 4(b) enters the object region, cells become more susceptible to large vibrations and, as a result, diffusion of the dosing liquid between the cells is conceivably promoted while preventing unnecessary dynamic action from being exerted on cell membrane. From the above, a vibrational frequency of injection pressure during the prescribed period of time Δt is favorably a frequency belonging to a prescribed frequency range associated with a natural frequency of a component (cells or the like) of the object region.

Moreover, the total amplitude of each of the vibration elements S2 to S4 other than the first vibration element S1 in the pressure vibration in the injection pressure transition in the prescribed period of time Δt is favorably equal to or lower than a total amplitude value (for example, 15 MPa) that is used as a reference. As a result, unnecessary dynamic action on cell membrane due to an excessively large total amplitude can be avoided.

Furthermore, the present inventors have focused on the fact that, in the pressure transition indicating a damped vibration within the prescribed period of time Δt, the peak value of the first vibration element S1 is relatively higher than those of the second, third, and fourth vibration elements S2 to S4. When an initial rise of injection pressure is an instantaneous pressure vibration element such as the first vibration element S1 described above, a minute distortion is created between cell membranes, thereby enabling forces directly acting on the cell membranes themselves to be suppressed while effectively canceling out the bonding force created by the bonding means described earlier. In other words, an instantaneous variation in injection pressure by the first vibration element S1 conceivably exerts dynamic action on the object region so as to mutually shift cells and to make it easier for the dosing liquid to flow along interfaces between the cells, and enables unnecessary dynamic action applied to cell membranes themselves to be suppressed. However, since the dynamic action to the cell membranes themselves conceivably becomes non-negligible when the peak value P×1 of the first vibration element S1 is excessively large, in consideration of minimal invasiveness, a prescribed upper limit ratio is favorably set for the peak value P×1 based on peak values of other vibrations (for example, the peak value P×2 of the second vibration element S2 which is a next highest peak value). A ratio of the peak value P×1 to the peak value P×2 corresponds to the prescribed first ratio according to the present invention. Therefore, in the present embodiment, the ratio is a value larger than 1 and lower than the prescribed upper limit ratio.

Based on the above, minimal invasiveness of the dosing liquid during administration can be demonstrated based on the fact that, in a transition of pressure (injection pressure) of the dosing liquid entering the object region in an initial stage of administration, the injection pressure vibrates in a damped manner and a peak value of the initial first vibration element S1 in vibrations of the injection pressure becomes larger within a range of a ratio larger than 1 and smaller than the prescribed upper limit ratio relative to peak values of other vibration elements (S2 to S4). The present inventors do not consider both conditions to be requisites in the transition of injection pressure for realizing minimally invasive administration. Minimally invasive administration can conceivably be realized by adopting any one of the conditions. However, more preferable minimally invasive administration can be realized by adopting both conditions in the transition of injection pressure.

Once the prescribed period of time Δt lapses, combustion of the gas generating agent is started by a combustion product of the ignition charge. As a result, the injection pressure of the dosing liquid of which pressure vibration had generally converged rises once again and the peak value Py described earlier arrives. Due to such pressurization of the dosing liquid by the gas generating agent, the dosing liquid having entered the object region at minimal invasiveness can be further diffused inside the object region in an effective manner.

In addition, even when the purpose of administration by the administration apparatus 1 is to introduce a prescribed substance to the inside of cells, a minimally invasive introduction of the substance into the cells can be achieved by adopting at least one of the two conditions described above in an injection pressure transition. Conceivably, by making the peak value of a first vibration element which is an initial vibration element in a group of vibration elements within the prescribed period of time Δt a steep peak value or by setting a frequency of the group of vibration elements to a frequency that belongs to the prescribed frequency range described earlier, at the same time as the entry of dosing liquid into the object region, cell membranes are pressurized over a wide range by the dosing liquid at a faster rate than a propagation of distortion through cells inside the object region and gaps are temporarily created in the cell membranes and, as a result, the introduction of the substance into the cells in a minimally invasive manner is promoted.

Moreover, instead of an aspect in which the peak value of the first vibration element which is an initial vibration element in pressure vibration within the prescribed period of time Δt is set using a peak value of other vibration elements (for example, the second vibration element) as a reference as described above, the peak value of the first vibration element or a flow rate corresponding to the peak value may be set higher than reference injection pressure or a reference flow rate set independently of parameters of the pressure vibration in the prescribed period of time Δt. The reference injection pressure or the reference flow rate may be appropriately changed in accordance with an object region to be an object of administration. For example, when the object region is an organ such as a liver or the heart, according to the minimal invasiveness provided by the present invention, a prescribed substance can be administered to tissue that does not constitute blood vessels in the organ while minimizing the effect on the blood vessels in the organ. Administration with minimal invasiveness is particularly useful for organs that contain a large amount of blood vessels such as the livers. In consideration thereof, the reference injection pressure and the reference flow rate described above are set so that minimal invasiveness on the blood vessels in organs can be achieved.

The formation of an injection pressure transition which enables minimally invasive administration to be performed by the administration apparatus 1 will now be described with reference to Fig. 5. In Fig. 5, an abscissa represents elapsed time in milliseconds and an ordinate represents pressure in MPa. In addition, in Fig. 5, a line L1 represents a transition of pressure inside the through-hole 64 in the administration apparatus 1, a line L2 represents a pressure transition of the dosing liquid 320 stored inside the storage chamber 32, and a line L3 represents injection pressure of the dosing liquid 320. The transition of injection pressure represented by L3 can be obtained by dividing an injection force obtained according to the known measurement method (for example, the measurement method described in Japanese Patent Application Laid-open No. 2005-21640) described earlier by an area (a nozzle area) of the injection port 31a of the administration apparatus 1. Moreover, in Fig. 5, with respect to pressure values of the injection pressure transition of the dosing liquid 320, 50% values of original values are displayed so as to overlap with other pressure transitions (lines L1 and L2). Furthermore, the pressure inside the through-hole 64 is measured by installing a pressure sensor to a pressure measurement port provided so as to be connected to the through-hole 64, and pressure of the dosing liquid inside the storage chamber 32 is measured by installing a pressure sensor to a pressure measurement port provided so as to be connected to the storage chamber 32.

As is evident from the pressure transitions, initially, pressure inside the through-hole 64 rises abruptly as the ignition charge of the igniter 71 burns. As the pressure rises, the piston 5 is propelled so as to push the plunger 4, and the dosing liquid is pressurized via the plunger 4. The pressure of the dosing liquid inside the storage chamber 32 due to the pressurization rises somewhat earlier than a rising time of injection pressure as indicated by the line L2, but exhibits a transition that is more or less steep as or even steeper than the rise of the injection pressure, and a subsequent pressure vibration exhibits a damped vibration at generally the same period as the injection pressure transition and at a generally constant frequency. In other words, in the example shown in Fig. 5, the injection pressure transition reaches a converged state in the prescribed period of time Δt of approximately 1.5 milliseconds. In addition, since the dosing liquid pressurized in this manner is injected from the injection port 31a, a series of operations for propelling the piston and pressurization by the plunger which is started by the combustion of the ignition charge in the administration apparatus 1 constitutes a pressurization operation for forming the injection pressure transition shown in Fig. 4 and, therefore, the piston 5 and the plunger 4 correspond to the pressurization unit according to the present invention.

The prescribed substance that is administered into the object region by the administration apparatus 1 will now be further described. When the prescribed substance is a substance which undergoes a change in physical properties when pressurized inside a liquid medium, efficiency of introduction of the substance into the object region can be enhancing using a reaction of the change. For example, Japanese Patent Application Laid-open No. 2003-261777 discloses a technique of forming a particulate hydrogel by placing polyvinyl alcohol and a water-soluble natural product capable of hydrogen bonding under high pressure. Under high pressure, since a hydroxyl group, an amino group, and a carboxylic group of the natural product bond to generate a microscopic hydrogen-bonded assembly, a three-dimensional molecular structure changes and may enable the natural product to more readily pass through membranes. In consideration thereof, using this characteristic of membrane permeability improvement due to pressure, administering the natural product with the administration apparatus 1 enables the prescribed substance to reach the inside of the object region in an efficient manner. In addition, when pressurization causes a reaction related to the prescribed substance to accelerate, the substance after the reaction can be introduced into the object region.

### [Practical example 1]

A result of an experiment regarding vibration in an object region when using the administration apparatus 1 according to the present invention will now be described with reference to Figs. 6 and 7. In the vibration experiment, injection of a dosing liquid is performed in a state where the injection port 31a of the administration apparatus 1 is arranged directly above a polyacrylamide gel simulating an object region. By imaging a vertical vibration (a vibration in an injection direction of the dosing liquid) of the polyacrylamide gel with a camera when injecting the dosing liquid and applying image processing to visualize the vertical vibration, a displacement due to the vibration was detected.

A line L11 in Fig. 6 represents a pressure transition (an injection pressure transition) of the injected dosing liquid when the dosing liquid is injected by the administration apparatus 1. Since the pressure transition is the same as that shown in Fig. 4, a detailed description thereof will be omitted. Furthermore, a line L12 in Fig. 6 represents a pressure transition (an injection pressure transition) of an injection solution injected by a spring-driven needle-free injector (hereinafter, referred to as a "spring-driven injector") which uses an elastic force of a spring as a driving source as a comparative example of the vibration experiment. The injection pressure transition by a spring-driven injector will now be briefly described. Generally, with a spring-driven injector, a spring (an elastic body) arranged inside its main body is compressed in advance, and by transferring elastic energy accumulated in the spring to an injection solution, an injection of the injection solution is performed. Since the spring released from the compressed state subsequently repetitively expands and contracts due to inertia, a vibration of the spring is to be strongly reflected in injection pressure as shown in Fig. 6. Specifically, it can be recognized that vibrations with a relatively large pressure amplitude continue until a time point (around approximately 18 milliseconds) at which a peak value due to combustion of a gas generating agent arrives in the administration apparatus 1.

In addition, in the spring-driven injector, an amplitude (a total amplitude) of the pressure vibration that continues for a long period of time after a rise timing of pressure varies irregularly. For example, in a range from 7.5 milliseconds to 9 milliseconds on the abscissa, the amplitude of the pressure vibration increases with the passage of time. Therefore, with the spring-driven injector, an amplitude variation of pressure vibration similar to the damped vibration exhibited by the administration apparatus 1 cannot be observed.

Therefore, when comparing the administration apparatus 1 according to the present invention with the spring-driven injector as a comparative example, while a pressure vibration in an initial stage of administration reaches a converged state within an extremely short period of time such as the prescribed period of time Δt with the administration apparatus 1, a pressure vibration continues for a relatively long period of time with the spring-driven injector. In addition, compared to the pressure vibration according to the administration apparatus 1 of which a pressure amplitude decreases with the passage of time during the prescribed period of time Δt, while the pressure vibration of the spring-driven injector eventually converges, a pressure amplitude thereof irregularly increases and decreases during the convergence of the pressure vibration. As described above, it is evident that the administration apparatus 1 according to the present invention and the spring-driven injector according to the comparative example exhibit completely different characteristics with respect to a transition of injection pressure.

In addition, Fig. 7 shows a transition of displacement due to vibration of a polyacrylamide gel when administration or the like is performed with respect to the polyacrylamide gel as described above using the administration apparatus 1 and a spring-driven injector having different characteristics of injection pressure transition as described above. In Fig. 7, an abscissa represents elapsed time in milliseconds and an ordinate represents displacement of the gel. Moreover, the displacement is indicated in terms of the number of pixels on a screen during image processing. Therefore, an amplitude in a vibration waveform represented by a line L13 in Fig. 7 signifies an amount of displacement (a vibration amplitude) of the gel during administration by the administration apparatus 1 and, in a similar manner, an amplitude in a vibration waveform represented by a line L14 signifies an amount of displacement (a vibration amplitude) of the gel during injection by the spring-driven injector.

In this case, as shown in Fig. 6, although injection pressure is generally the same between the administration apparatus 1 and the spring-driven injector in an initial stage of injection of the dosing liquid or the like, as is obvious from a comparison between the transition of displacement represented by the line L13 and the transition of displacement represented by the line L14, the spring-driven injector exhibits a larger amount of displacement of the gel than the administration apparatus 1. In particular, it is recognized that, in the initial stage of injection of the dosing liquid or the like, the amount of displacement of the administration apparatus 1 is generally constant but the amount of displacement of the spring-driven injector exhibits extremely large variations. This suggests that, after entering the object region, the injection solution injected by the spring-driven injector undergoes a significant displacement inside the object region and, as a result, the injection solution may possibly exert an unnecessary dynamic action on the object region. On the other hand, it is also suggested that, even after entering the object region, an amount of displacement of the dosing liquid injected by the administration apparatus 1 inside the object region is kept small and, as a result, a situation where an unnecessary dynamic action is exerted on the object region can be avoided. Although a polyacrylamide gel and tissue and cells of a living organism are structurally different from each other, since the dynamic action exerted by the administration apparatus 1 on the object region is extremely small as shown in Fig. 7, it is highly expected that preferable minimal invasiveness is exhibited even during an administration to an object living organism.

### [Practical example 2]

Experimental conditions and experimental results of an intradermal administration experiment to rabbit in vivo by the administration apparatus 1 according to the present invention and a needled injector according to a comparative example are described below. Moreover, an object of the following experimental conditions is to confirm, when an inflammatory substance is administered to the intradermal layer which is skin tissue of the rabbit in vivo, a presence or absence of an inflammatory response (formation of erythema and eschar, and formation of edema) for each elapsed time (after 24 hours, 48 hours, and 72 hours). In addition, the injection of an injection solution with the needled injector was performed at a constant pressurization rate.

### <Experimental conditions>

### (Administration object)

16-week-old male Std: JW/CSK rabbit, already shaved and smooth skin-verified, was used.

### (Dosing liquid and injection solution)

An aqueous solution of the following inflammatory substance was used as the dosing liquid and the injection solution.

Inflammatory substance: acetic acid, diluted to a predetermined concentration with distilled water, dose 100 µl

### <Experimental results>

Experimental results are shown in Tables 1 and 2 presented below. Definitions of numerical values representing the experimental results in the tables with respect to each of erythema and eschar formation and edema formation are as follows.

**(Erythema and eschar formation)**

| | |
|---|---|
| 0 | No erythema |
| 1 | Very slight erythema (barely perceptible erythema) |
| 2 | Slight erythema (clearly perceptible erythema) |
| 3 | Moderate to severe erythema |
| 4 | Severe erythema to eschar formations |

**(Edema formation)**

| | |
|---|---|
| 0 | No edema |
| 1 | Very slight edema (barely perceptible edema) |
| 2 | Slight edema (edema with edges perceptible due to definite raising) |
| 3 | Moderate edema (raised approximately 1 mm) |
| 4 | Severe edema (raised 1 mm or more and extending beyond the area of exposure) |

Experiment results with respect to rabbit in vivo are shown in Table 1.

**[Table 1]**

| Acetic acid concentration | Injector 1 | | | | | | Comparative example (needled injector) | | | | | |
|---|---|---|---|---|---|---|---|---|---|---|---|---|
| | Erythema etc. | | | Edema | | | Erythema etc. | | | Edema | | |
| | 24h | 48h | 72h | 24h | 48h 72h | | 24h | 48h | 72h | 24h | 48h | 72h |
| 0.25% | 1 | 1 | 1 | 0 | 0 | 0 | 1 | 1 | 1 | 0 | 0 | 0 |
| 0.5% | 1 | 1 | 1 | 0 | 0 | 0 | 1 | 1 | 1 | 0 | 0 | 0 |
| 1.0% | 1 | 1 | 1 | 0 | 0 | 0 | 1 | 1 | 1 | 1 | 1 | 1 |
| 1.5% | 1 | 1 | 1 | 0 | 0 | 0 | 1 | 1 | 1 | 2 | 2 | 2 |
| 2.0% | 1 | 1 | 1 | 1 | 1 | 1 | 1 | 1 | 1 | 3 | 3 | 2 |

As is evident from the comparison in the table presented above, according to the administration apparatus 1, an inflammatory response in intradermal cells due to the inflammatory substance contained in the dosing liquid is effectively suppressed as compared to the comparative example. This conceivably indicates that the inflammatory substance is successfully administered and diffused into the intradermal layer without invading the intradermal cells. As described above, according to the present invention, administration which suppresses invasiveness to cells and which diffuses a dosing liquid over a wide range of skin tissue can be realized.

### [Practical example 3]

Experimental conditions and experimental results of an administration experiment to porcine abdominal skin tissue by the administration apparatus 1 according to the present invention and a needled injector and a spring-driven injector (a needle-free injector) according to a comparative example are described below. Moreover, an object of the following experimental conditions is to administer an MRI contrast medium to porcine abdominal skin tissue and check a state of entry of the MRI contrast medium inside the porcine abdominal skin tissue based on an image captured by an MRI apparatus.

### <Experimental conditions>

### (Administration apparatus 1)

Flow rate: 202 m/s (corresponding to injection pressure of approximately 40 MPa)
Nozzle diameter: Ø 0.17 mm

### (Needled injector of comparative example)

The injection of an injection solution with the needled injector was performed at a constant pressurization rate.
Needle: 27 G

### (Spring-driven injector of comparative example)

Flow rate: 205 m/s (corresponding to injection pressure of approximately 40 MPa)
Nozzle diameter: Ø 0.17 mm

### (Administration object)

Porcine skin tissue collected from abdominal area of a 3-month old female pig (approximately 50 kg)

### (Dosing liquid and injection solution)

Contrast agent Magnevist manufactured by Bayer Yakuhin, Ltd. was diluted to be used as a dosing liquid and an injection solution. In imaging by an MRI apparatus, contrast of an MRI image peaks when concentration of a contrast agent in a dosing liquid or the like is further diluted. In addition, the dose was 100 µl.

### <Experimental results>

Fig. 8 shows MRI images of a state of entry of a dosing liquid or the like when administration or the like is performed with respect to porcine abdominal skin tissue by each apparatus. Magnifications of the respective MRI images are the same. (1) in Fig. 8 is an MRI image of administration by the administration apparatus 1. (2) in Fig. 8 is an MRI image of an injection by the needled injector according to the comparative example, and (3) in Fig. 8 is an MRI image of an injection by the spring-driven injector according to the comparative example. As is evident from each MRI image, when performing administration by the administration apparatus 1, in a relatively shallow portion (portions with depths up to around 10 mm) during a process of the dosing liquid entering the skin tissue from an entrance position thereof (a process of entering a deep side from an entry side in the image), a contrast of portions through which the dosing liquid had passed is clearly stronger than in the cases of the needled injector and the spring-driven injector. This means that, after being injected into skin tissue, the contrast agent contained in the dosing liquid diffuses in a vicinity of a flow path in a shallow portion of the skin tissue and, as a result, the contrast agent is further diluted to around 0.1 mmol/kg. In addition, in the diffusion, since a volume change of the skin tissue was not recognized, it is surmised that the dosing liquid moistens between cells and, at the same time, diffuses into the tissue so as to also enter the cells, thereby achieving minimally invasive administration.

On the other hand, as shown in (3) in Fig. 8, in the case of the needled injector according to the comparative example, the injection solution simply accumulates in a state of a liquid reservoir in a relatively shallow portion and cannot be described as being widely diffused in the skin tissue as was the case of the administration apparatus 1. In addition, in the case of the spring-driven injector according to the comparative example, the contrast by the contrast agent in a shallow portion from an entrance position has significantly declined as compared to the case of the administration apparatus 1 and, at the same time, a flow path diameter has become narrower. Specifically, in the result of the administration apparatus 1 shown in (1) in Fig. 8, a diffusion diameter in a vicinity of a flow path in a relatively shallow portion is approximately 2.2 mm, and a separate experiment using the administration apparatus 1 with different injection pressure resulted in the diffusion diameter expanding to approximately 4.4 mm. On the other hand, in the result of the spring-driven injector shown in (2) of Fig. 8, the diffusion diameter in a vicinity of a flow path in a relatively shallow portion is approximately 1.3 mm. In addition, in the case of the spring-driven injector, unlike in the case of the administration apparatus 1, the injection solution having entered the skin tissue penetrated the object region and was unable to reach an appropriate injection depth. Given that flow rates of the injected dosing liquid and the like are roughly the same and nozzle diameters are also the same in both the administration apparatus 1 and the spring-driven injector, in the case of the spring-driven injector, it is surmised that diffusion of the injection solution in a relatively shallow portion is not promoted and a large part of the injection solution simply proceeds to a deep side. Furthermore, it is shown that, even with the administration apparatus 1, a degree of diffusion of the dosing liquid in a relatively shallow portion can be adjusted by varying administration conditions.

Such a distinct difference between the administration apparatus 1 and the needled injector and the spring-driven injector is conceivably due to a presence or absence of the characteristic injection pressure transition of a dosing liquid according to the present invention described heretofore. In other words, with the dosing liquid injected by the administration apparatus 1, it is evident that invasiveness of the dosing liquid to cells is suppressed and the dosing liquid is injected so as to enable the dosing liquid to be diffused over a wide range of tissue by entering the porcine abdominal skin tissue with the pressure transition shown in Fig. 4.

### [Practical example 4]

Experimental conditions and experimental results of an administration experiment to rat skin tissue by the administration apparatus 1 according to the present invention and a needled injector according to a comparative example are described below. Moreover, an object of the following experimental conditions is to check a state of diffusion of a dosing liquid inside the rat skin tissue.

### <Experimental conditions>

### (Administration apparatus 1)

Injection pressure: 10.9 MPa
Nozzle diameter: Ø 0.11 mm

### (Needled injector of comparative example)

The injection of a dosing liquid with the needled injector was performed at a constant pressurization rate.
Needle: 27 G

### (Administration object)

SD rat skin tissue of a 10-week-old male rat.

### (Dosing liquid)

The dosing liquid was India ink and a dose thereof was 10 µl.

### <Experimental results>

An upper part (1) in Fig. 9 shows a micrograph of a rat skin tissue section at a location where the administration experiment had been performed by the administration apparatus 1, and a lower part (2) shows a micrograph of a rat skin tissue section at a location where an injection had been performed by the needled injector. Magnifications of both micrographs are the same, but administration objects corresponding to the respective micrographs represent different individuals. Moreover, while the diagram shows pools of India ink enclosed by solid lines, it is to be understood that the solid lines representatively indicate a part of the pools of India ink and do not indicate all of the pools of India ink. The result of the administration by the administration apparatus 1 shows that India ink that is the dosing liquid has been widely diffused in skin tissue. In this state, since India ink is diffused in the skin tissue as pools of extremely small amounts of India ink, it is readily recognized that a preferable diffusion of the dosing liquid is achieved. In addition, given that India ink has not reached muscular tissue or a vicinity of a boundary between muscular tissue and skin tissue, it is evident that India ink is widely diffused in an extremely shallow portion in the skin tissue. On the other hand, the result of the injection by the needled injector shown in the lower part (2) shows that the injected India ink is accumulated in a vicinity of a boundary between muscular tissue and skin tissue and a large liquid reservoir is formed. In addition, a part of the India ink has reached the inside of muscular tissue. As a result, it is evident that the needled injector is unable to preferably diffuse the India ink in skin tissue and, at the same time, retaining the India ink in relatively shallow skin tissue is not easy using the needled injector.

Such a distinct difference between the administration apparatus 1 and the needled injector is conceivably due to a presence or absence of the characteristic injection pressure transition of a dosing liquid according to the present invention described heretofore. In other words, with the dosing liquid injected by the administration apparatus 1, it is evident that invasiveness of the dosing liquid to cells is suppressed and the dosing liquid is injected so as to enable the dosing liquid to be diffused over a wide range of tissue by entering the rat skin tissue with the pressure transition shown in Fig. 4.

### <Other embodiments>

The administration apparatus 1 according to the present invention can also be preferably utilized to introduce a biologically-derived substance such as genes. For example, for the purpose of regenerative medicine with respect to humans, cultured cells, stem cells, or the like can be seeded to cells or scaffold tissue/scaffolds to be used as administration objects. For example, as described in Japanese Patent Application Laid-open No. 2008-206477, cells which can be appropriately determined by a person skilled in the art in accordance with a site of implantation and a purpose of recellularization such as endothelial cells, endothelial progenitor cells, bone marrow cells, preosteoblasts, chondrocytes, fibroblasts, skin cells, muscle cells, liver cells, kidney cells, intestinal cells, stem cells, and any other cells considered in the field of regenerative medicine can be administered by the administration apparatus 1.

In addition, the administration apparatus 1 according to the present invention can also be used to deliver DNA or the like to cells, scaffold tissue/scaffolds, and the like such as described in Japanese Translation of PCT Application No. 2007-525192. In this case, since the use of the administration apparatus 1 according to the present invention enables an effect on the cells, scaffold tissue/scaffolds, and the like to be suppressed as compared to delivery using a needle, it can be said that the use of the administration apparatus 1 according to the present invention is more favorable.

Furthermore, the administration apparatus 1 according to the present invention can also be preferably used when directly delivering various genes, cancer suppressor cells, a lipid envelope, or the like to object tissue or when administering an antigen gene in order to improve immunity to pathogens. Moreover, the administration apparatus 1 can also be used in various fields of disease treatment (the fields described in Japanese Translation of PCT Application No. 2008-508881, Japanese Translation of PCT Application No. 2010-503616, and the like), the field of immunological medicine (the field described in Japanese Translation of PCT Application No. 2005-523679), and the like, and fields in which the administration apparatus 1 is usable are not intentionally limited.

In the embodiments heretofore described, administration with minimal invasiveness to an object region is realized by performing injection of a dosing liquid using an ignition charge or a combination of an ignition charge and a gas generating agent as a power source and adjusting injection pressure to a pressure transition such as that shown in Fig. 4. In order to achieve such minimally invasive administration, a transition of injection pressure of the dosing liquid is extremely important as described earlier. In other words, any type of driving source for injecting the dosing liquid can be used as long as the characteristic injection pressure transition of the present invention described earlier can be realized. For example, a piezoelectric actuator such as a piezoelectric element, an electrical actuator such as an ultrasonic wave, a pneumatic actuator such as compressed gas, a hydraulic actuator using pressure created by boiling and expansion of a liquid by laser, and the like can be used as a driving source for pressurizing the dosing liquid.

In addition, the characteristic injection pressure transition of a dosing liquid according to the present invention involves a steep rise of pressure as well as a damped vibration in a prescribed period of time Δt as described earlier. In order to effectively form the damped vibration, a gas layer constituted by air or inert gas may be included in the dosing liquid insofar as the gas layer does not interfere with the administration to the object region. The inclusion of such a gas layer conceivably enables an effective damped vibration to be formed when pressurization is performed with combustion energy of an ignition charge due to an elastic compressive deformation of the air layer. As a result, realization of minimally invasive administration is expected.

In addition, while the piston 5 is adopted as a configuration (hereinafter, also referred to as a "transmission configuration") which transmits combustion energy of the ignition charge to the dosing liquid in the embodiments heretofore described, as an alternative mode, energy may be transmitted by a mechanical element such as a diaphragm or a ram. In particular, as the diaphragm, a membrane-like form made of an elastic material or a plate-like form made of a metallic material can be adopted. Adopting a diaphragm as the transmission configuration enables a combustion space in which combustion of an ignition charge is performed and an arrangement space in which a dosing liquid is arranged to be separated from each other by the diaphragm. Therefore, it is also possible to prevent a combustion product of the combustion of the ignition charge from being mixed into the dosing liquid. Moreover, the transmission configuration is favorably arranged in a similar manner to the piston 5 in a single row between the plunger 4 which pushes the dosing liquid for injection and the drive unit 7. Such an arrangement realizes pressurization of the dosing liquid in an efficient manner and thereby enables the characteristic injection pressure transition of a dosing liquid according to the present invention described earlier to be achieved.

Furthermore, in the embodiments heretofore described, the dosing liquid is stored in the storage chamber 32 formed in the body 30 of the syringe section 3. Examples of a material of the body 30 include glass, a resin, and a metal. When combustion energy created by combustion of the ignition charge is transmitted to the dosing liquid inside the storage chamber 32 via the piston 5, although pressurization via the plunger 4 conceivably mainly contributes to the formation of the characteristic injection pressure transition of a dosing liquid according to the present invention, the transition may possibly be also formed due to vibration of the piston 5 having received the combustion energy being transmitted to the dosing liquid via the body 30. In consideration thereof, the material of the body 30 may be determined from such a perspective.

In addition, while a pressurization structure in which injection of the dosing liquid from the body 30 of the syringe section 3 is performed using pressurization by the plunger 4 via the piston 5 is adopted in the embodiments heretofore described, other pressurization structures may be adopted as long as the characteristic injection pressure transition of a dosing liquid according to the present invention described earlier is realized. For example, in another useful pressurization structure, the dosing liquid is stored in advance in a flexible container that deforms when pressurized from the outside and, when the container is pressurized by administration energy from the drive unit 7, the container deforms to cause the dosing liquid inside the container to be injected outward.

Furthermore, reference will now be made to an administration apparatus in a mode that differs from those of the embodiments heretofore described as an administration apparatus to which the present invention can be applied. For example, the present invention can be preferably applied to apparatuses used in the field of cell processing. While a prescribed substance can also be introduced into a cell in the modes described above, the present invention can be applied to form an introduction apparatus for introducing a biologically-derived substance such as genes into a cell. In this case, the biologically-derived substance to be introduced is included in a liquid, and the liquid is pressurized and injected to an object cell. In addition, a minimally invasive introduction of the substance to the cell is realized by adjusting injection pressure of the liquid to the characteristic injection pressure transition according to the present invention described earlier. Moreover, when introducing the substance, the substance may be simultaneously introduced into a cell from a plurality of paths.

Examples of an alternative administration apparatus to which the present invention can be applied include a catheter apparatus. The catheter apparatus is an apparatus which has a catheter unit capable of entering a living organism and which administers a desired drug solution or the like to the living organism from a tip section of the catheter unit. The present invention can be applied to a configuration for the administration of a drug solution from the tip section of the catheter unit. In other words, in a state where the catheter unit has entered inside the living organism, by controlling injection pressure of a drug solution to match the characteristic injection pressure transition according to the present invention described earlier when administering the drug solution from the tip section of the catheter unit, a minimally invasive administration of the drug solution to a prescribed site of an object region (for example, an internal organ such as the heart or a liver) to which the drug solution is to be administered can be achieved.

As an alternative method of administration using a catheter apparatus to which the present invention is applied, when injecting a drug solution from the tip section of the catheter unit, the drug solution can be administered in the form of successive droplets or, in other words, in a state of a high-speed pulse. According to such an administration mode, the drug solution can be administered in a mode of a finer aerosol and, conceivably, administration of the drug solution with even lower invasiveness can be realized. Such pulsification of a drug solution can be achieved by causing an entire reservoir storing the drug solution or a part of the reservoir to repetitively expand and contract at a high rate. In doing so, the expansion and contraction of the reservoir are repeated so that a transition of injection pressure of the drug solution to be injected matches the characteristic injection pressure transition according to the present invention described earlier. The expansion and contraction of the reservoir can be repeated using a pulse vibration generation apparatus which electrically generates vibrations together with a mechanical configuration capable of transmitting or amplifying the pulse vibration.

### [Reference Signs List]

- 1: Administration apparatus
- 2: Housing
- 3: Syringe section
- 4: Plunger
- 5: Piston
- 6: Administration apparatus main body
- 7: Drive unit
- 8: Button
- 9: Battery
- 10: Apparatus assembly
- 10A, 10B: Sub-assembly
- 31: Nozzle section
- 32: Storage chamber
- 44: Rod section
- 53: Pressing column section
- 54: Storage hole
- 64: Through-hole
- 71: Igniter

## Claims

1. An administration apparatus (1) which administers a dosing liquid (320) containing a prescribed substance to an object region, the administration apparatus comprising:
a main body (6) comprising a through-hole (64);
a storage unit (32) which stores the dosing liquid (320);
a drive unit (7) which imparts administration energy, wherein the drive unit comprises an igniter (71);
a pressurization unit (4,5) comprising a piston (5) configured to be pressurized by a combustion product generated by the igniter (71) and to slide inside the through-hole (64), such that the pressurization unit (4,5) pressurizes the dosing liquid (320) stored in the storage unit (32) with the administration energy in the drive unit (7); and
an injection unit which injects the dosing liquid (320) having been pressurized by the pressurization unit (4,5) to the object region through an injection port (31a), wherein
the pressurization unit is configured to pressurize the dosing liquid (320) so that, in a pressure transition (L3)of the dosing liquid (320) at the injection port (31a) in response to imparting the administration energy, a vibration in the pressure transition reaches a converged state within a prescribed period of time (Δt) from a rising time of pressure (T0), wherein the vibration in the pressure transition comprises a first vibration element (S1) followed by successive second, third and fourth vibration elements (S2, S3, S4), wherein a ratio of a peak value (P×1) of the first vibration element (S1) in the vibration within the prescribed period of time to a peak value (Px2) of subsequent vibration elements (S2, S3, S4) other than the first vibration element is higher than 1 and equal to or lower than a prescribed first ratio;
wherein the drive unit (7) includes at least a high-energy substance and generates the administration energy by burning the high-energy substance;
wherein the first vibration element (S1) is a vibration that reaches pressure equal to or higher than 10 MPa and up to approximately 45 MPa that is the peak value (P×1) within 0.5 msec from the rising time(T0);
wherein within the prescribed period of time (Δt) from the rising time (T0), pressure of the dosing liquid (320) reaches the converged state while vibrating in a damped manner at a frequency belonging to a prescribed frequency range from 500 to 10000 Hz;
wherein a total amplitude of the second vibration element (S2) is approximately 10 MPa, and a total amplitude of the third and fourth vibration elements (S3, S4) included in the vibration in the prescribed period of time (Δt) decreases with the passage of time such that the pressure transition becomes a damped vibration with the passage of time;
wherein the prescribed period of time (Δt) is a period of 2 msec or less from the rising time (T0).

2. The administration apparatus (1) according to claim 1, wherein
the prescribed period of time (Δt) is a period of 1.5 msec or less from the rising time (T0).

3. The administration apparatus (1) according to claim 1, further comprising
a gas generating agent which generates prescribed gas by combustion and which is arranged at a position exposed to combustion products generated by the combustion of the high-energy substance, wherein
the pressurization unit (4,5) further pressurizes the dosing liquid (320) by energy created by the combustion of the gas generating agent in a vicinity of a time at which the prescribed period of time (Δt) has elapsed from the rising time (T0) of pressure of the dosing liquid (320).

4. The administration apparatus (1) according to claim 3, wherein
when the administration energy is defined as first administration energy, and
energy created by the combustion of the gas generating agent is defined as second administration energy,
a peak value (Py) in a pressure transition of the dosing liquid (320) by the second administration energy is equal to or higher than a peak value (Px2) in a pressure transition excluding the first vibration element (S1) in the pressure transition of the dosing liquid (320) by the first administration energy.

5. The administration apparatus (1) according to any one of claims 1 to 4, wherein
the administration apparatus is an apparatus which administers the dosing liquid (320) to the object region from the injection port (31a) without involving an introduction unit.

## Patentansprüche

1. Verabreichungsvorrichtung (1), die eine Dosierflüssigkeit (320), die eine vorgegebene Substanz enthält, in einen Objektbereich verabreicht, wobei die Verabreichungsvorrichtung folgendes umfasst:
einen Hauptkörper (6) mit einer Durchgangsbohrung (64);
eine Speichereinheit (32), die die Dosierflüssigkeit (320) speichert;
eine Antriebseinheit (7), die Verabreichungsenergie überträgt, wobei die Antriebseinheit einen Zünder (71) umfasst;
eine Druckbeaufschlagungseinheit (4, 5), die einen Kolben (5) umfasst, der so konfiguriert ist, dass er durch ein von dem Zünder (71) erzeugtes Verbrennungsprodukt mit Druck beaufschlagt wird und innerhalb der Durchgangsbohrung (64) gleitet, so dass die Druckbeaufschlagungseinheit (4, 5) die in der Speichereinheit (32) gespeicherte Dosierflüssigkeit (320) mit der Verabreichungsenergie in der Antriebseinheit (7) unter Druck setzt; und
eine Injektionseinheit, die die von der Druckbeaufschlagungseinheit (4, 5) unter Druck gesetzte Dosierflüssigkeit (320) durch eine Injektionsöffnung (31a) in den Objektbereich injiziert, wobei
die Druckbeaufschlagungseinheit so konfiguriert ist, dass sie die Dosierflüssigkeit (320) mit Druck beaufschlagt, so dass in einem Druckübergang (L3) der Dosierflüssigkeit (320) an der Injektionsöffnung (31a) als Reaktion auf das Aufbringen der Verabreichungsenergie eine Schwingung in dem Druckübergang innerhalb einer vorgegebenen Zeitspanne (Δt) ab einer Druckanstiegszeit (T0) einen konvergierten Zustand erreicht, wobei die Vibration in dem Druckübergang ein erstes Vibrationselement (S1), gefolgt von aufeinanderfolgenden zweiten, dritten und vierten Vibrationselementen (S2, S3, S4), umfasst, wobei ein Verhältnis eines Höchstwertes (Px1) des ersten Vibrationselements (S1) in der Vibration innerhalb der vorgegebenen Zeitspanne zu einem Höchstwert (Px2) von nachfolgenden Vibrationselementen (S2, S3, S4), die nicht das erste Vibrationselement sind, größer als 1 und gleich oder niedriger als ein vorgegebenes erstes Verhältnis ist;
wobei die Antriebseinheit (7) mindestens eine hochenergetische Substanz enthält und die Verabreichungsenergie durch Verbrennen der hochenergetischen Substanz erzeugt;
wobei das erste Vibrationselement (S1) eine Vibration ist, die einen Druck gleich oder höher als 10 MPa und bis zu ungefähr 45 MPa erreicht, was der Höchstwert (Px1) innerhalb von 0,5 msec ab der Anstiegszeit (T0) ist;
wobei innerhalb der vorgegebenen Zeitspanne (Δt) ab der Anstiegszeit (T0) der Druck der Dosierflüssigkeit (320) den konvergierten Zustand erreicht, während er in einer gedämpften Weise mit einer Frequenz schwingt, die zu einem vorgegebenen Frequenzbereich von 500 bis 10000 Hz gehört;
wobei eine Gesamtamplitude des zweiten Vibrationselements (S2) etwa 10 MPa beträgt und eine Gesamtamplitude des dritten und vierten Vibrationselements (S3, S4), die in der Vibration in der vorgegebenen Zeitspanne (Δt) enthalten sind, mit dem Lauf der Zeit abnimmt, so dass der Druckübergang mit dem Lauf der Zeit zu einer gedämpften Vibration wird;
wobei die vorgeschriebene Zeitspanne (Δt) eine Zeitspanne von 2 msec oder weniger ab der Anstiegszeit (T0) ist.

2. Verabreichungsvorrichtung (1) nach Anspruch 1, wobei
die vorgegebene Zeitspanne (Δt) eine Zeitspanne von 1,5 msec oder weniger ab der Anstiegszeit (T0) ist.

3. Verabreichungsvorrichtung (1) nach Anspruch 1, ferner umfassend
ein Gaserzeugungsmittel, das durch Verbrennung vorgegebenes Gas erzeugt und das an einer Position angeordnet ist, die Verbrennungsprodukten, welche durch die Verbrennung der hochenergetischen Substanz erzeugt werden, ausgesetzt ist, wobei
die Druckbeaufschlagungseinheit (4, 5) die Dosierflüssigkeit (320) durch Energie, die durch die Verbrennung des Gaserzeugungsmittels erzeugt wird, in der Nähe eines Zeitpunkts, zu dem die vorgegebene Zeitspanne (Δt) ab der Druckanstiegszeit (T0) der Dosierflüssigkeit (320) verstrichen ist, weiter unter Druck setzt.

4. Verabreichungsvorrichtung (1) nach Anspruch 3, wobei
wenn die Verabreichungsenergie als erste Verabreichungsenergie definiert ist, und
Energie, die durch die Verbrennung des Gaserzeugungsmittels erzeugt wird, als zweite Verabreichungsenergie definiert ist,
ein Höchstwert (Py) in einem Druckübergang der Dosierflüssigkeit (320) durch die zweite Verabreichungsenergie gleich oder höher ist als ein Höchstwert (Px2) in einem Druckübergang ohne das erste Schwingungselement (S1) im Druckübergang der Dosierflüssigkeit (320) durch die erste Verabreichungsenergie.

5. Verabreichungsvorrichtung (1) nach einem der Ansprüche 1 bis 4, wobei
die Verabreichungsvorrichtung eine Vorrichtung ist, welche die Dosierflüssigkeit (320) aus der Injektionsöffnung (31a) ohne Einbeziehung einer Einbringungseinheit in den Objektbereich verabreicht.

## Revendications

1. Appareil applicateur (1) pour administrer un liquide doseur (320) contenant une substance prescrite à une région objet,
l'appareil applicateur comprenant :
- un corps principal (6) avec un orifice traversant (64),
- une unité de stockage (32) qui reçoit le liquide doseur (320),
- une unité d'entraînement (7) qui fournit l'énergie d'application,
l'unité d'entraînement comprenant un déclencheur (71),
- une unité de mise en pression (4, 5) comprenant un piston (5) configuré pour être mis en pression par le produit de combustion généré par le déclencheur (71) et glisser dans l'orifice traversant (64) de façon que l'unité de mise en pression (4, 5) mette en pression le liquide doseur (320) contenu dans l'unité de stockage (32) avec l'énergie d'application de l'unité d'entraînement (7), et
- une unité d'injection qui injecte le liquide doseur (320) mis en pression par l'unité de mise en pression (4, 5) dans la région objet par un orifice d'injection (31a),
* l'unité de mise en pression étant configurée pour mettre en pression le liquide doseur (320) pour que dans une transition de pression (L3) du liquide doseur (320) au port d'injection (31a), en réponse à la mise en œuvre de l'énergie d'application, une vibration dans la transition de pression atteint un état de convergence dans une période de temps prescrite (Δt) à partir du temps de montée en pression (TO),
* la vibration dans la transition de pression comprenant un premier élément de vibration (S1) suivi par un second, un troisième et un quatrième élément de vibration successif (S2, S3, S4),
* le rapport entre la valeur de crête (P×1) du premier élément de vibration (S1) dans la vibration dans la période prescrite jusqu'à une valeur de crète (P×2) des éléments de vibration suivants (S2, S3, S4) autres que le premier élément de vibration est supérieur à 1 et égal ou inférieur au premier rapport prescrit,
* l'unité d'entraînement (7) comprenant au moins une substance à forte énergie et générant l'énergie d'application par la combustion de la substance à forte énergie,
* le premier élément de vibration (S1) étant une vibration qui atteint une pression égale ou supérieure à 10 MPa et jusqu'à approximativement 45 MPa qui est la valeur de (P×1) dans 0,5 msec à partir du temps de montée (TO),
* dans la période de temps prescrite (Δt) à partir du temps de montée (TO), la pression de la dose de liquide (320) atteint l'état de convergence pendant que la vibration est amortie à une fréquence dans une plage de fréquences prescrites comprise entre 500 et 10000 Hz,
* l'amplitude totale du second élément de vibration (S2) étant approximativement égale à 10 MPa et l'amplitude totale du troisième et du quatrième élément de vibrations (S3, S4) contenu dans la vibration pendant la période de temps prescrite (Δt) diminuant avec le temps de façon que la transition de pression soit une vibration amortie en fonction du temps,
* la période de temps prescrite (Δt) étant une période de 2 msec ou moins à partir du temps de montée (T0).

2. Appareil applicateur (1) selon la revendication 1,
dans lequel
la période de temps prescrite (Δt) est une période de 1,5 msec ou moins à partir du temps de montée (T0).

3. Appareil applicateur (1) selon la revendication 1,
comprenant en outre :
- un agent générant du gaz qui génère le gaz prescrit par la combustion et qui est organisé dans une position exposée aux produits de la combustion de la substance à forte énergie,
* l'unité de mise en pression (4, 5) met en outre en pression le liquide doseur (320) en créant de l'énergie par la combustion de l'agent générant du gaz près de l'instant auquel se termine la période de temps prescrite (Δt), à compter du temps de montée (T0) de la pression d'un liquide doseur (320).

4. Appareil applicateur (1) selon la revendication 3,
dans lequel
si l'énergie d'administration est définie comme première énergie d'administration, et
si l'énergie créée par la combustion de l'agent générant le gaz est définie comme seconde énergie d'administration,
une valeur de (Py) dans la transition de pression d'un liquide doseur (320) par la seconde énergie d'application est égale ou supérieure à une valeur de (Px2) dans la transition de pression excluant le premier élément de vibration (S1) dans la transition de pression d'un liquide doseur (320) par la première énergie d'administration.

5. Appareil applicateur (1) selon l'une quelconque des revendications 1 à 4,
dans lequel
l'appareil applicateur est un appareil qui administre le liquide doseur (320) dans la région objet à partir de l'orifice d'injection (31a) sans utiliser d'unité d'introduction.
